(19) **Europäisches Patentamt / European Patent Office / Office européen des brevets**

(11) **EP 3 895 890 B1**

(12) **EUROPEAN PATENT SPECIFICATION**

(45) Date of publication and mention of the grant of the patent:
**03.07.2024 Bulletin 2024/27**

(21) Application number: **19895351.5**

(22) Date of filing: **09.12.2019**

(51) International Patent Classification (IPC):
**B32B 27/00** *(2006.01)*  **C08G 81/00** *(2006.01)*
**A61L 15/26** *(2006.01)*  **A61L 15/28** *(2006.01)*
**A61L 15/32** *(2006.01)*  **A61L 15/42** *(2006.01)*
**A61L 15/44** *(2006.01)*  **A61L 15/58** *(2006.01)*
**A61L 15/64** *(2006.01)*

(52) Cooperative Patent Classification (CPC):
(C-Sets available)
**A61L 24/0036; A61L 15/425; A61L 15/585;**
**A61L 15/64; A61L 24/0042; A61L 24/043;**
**A61L 24/108**                                (Cont.)

(86) International application number:
**PCT/JP2019/048068**

(87) International publication number:
**WO 2020/122007 (18.06.2020 Gazette 2020/25)**

(54) **TWO-REACTANT SHEET-FORM TISSUE-ADHESIVE-REINFORCING MATERIAL**

BLATTFÖRMIGES, GEWEBEADHÄSIVES VERSTÄRKENDES MATERIAL MIT ZWEI REAKTIONSMITTELN

MATÉRIAU DE RENFORCEMENT D'ADHÉSIF DE TISSU EN FORME DE FEUILLE À DEUX RÉACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.12.2018 JP 2018234952**

(43) Date of publication of application:
**20.10.2021 Bulletin 2021/42**

(73) Proprietor: **BMG INCORPORATED**
**Kyoto-shi,**
**Kyoto 601-8023 (JP)**

(72) Inventors:
• **TANIDA, Shuhei**
**Kyoto-shi, Kyoto 601-8023 (JP)**
• **FUJIMURA, Motoki**
**Kyoto-shi, Kyoto 601-8023 (JP)**
• **KOJITANI, Yusuke**
**Kyoto-shi, Kyoto 601-8023 (JP)**
• **OSADA, Shinichi**
**Kyoto-shi, Kyoto 601-8023 (JP)**

• **HYON, Woogi**
**Kyoto-shi, Kyoto 601-8023 (JP)**
• **HYON, Suong-Hyu**
**Kyoto-shi, Kyoto 601-8023 (JP)**

(74) Representative: **Global IP Europe**
**Patentanwaltskanzlei**
**Pfarrstraße 14**
**80538 München (DE)**

(56) References cited:
**EP-A1- 2 100 628           WO-A1-2006/080523**
**WO-A1-2008/066182      JP-A- 2004 520 124**
**JP-A- 2004 521 115       JP-A- 2014 518 250**
**JP-A- 2014 533 988       JP-A- 2016 063 919**
**JP-A- 2017 202 033       JP-A- 2017 500 113**
**US-A1- 2008 319 101**

• **Hyon, Suong-Hyu: "Medical Adhesive using the Food Additives", Journal of The Adhesion Society of Japan, vol. 49, no. 8, 1 August 2013 (2013-08-01) , pages 293-304, XP009522152, ISSN: 0916-4812, DOI: 10.11618/adhesion.49.293**

EP 3 895 890 B1

(52) Cooperative Patent Classification (CPC): (Cont.)

C-Sets
**A61L 15/585, C08L 1/02;**
**A61L 15/585, C08L 89/00;**
**A61L 24/043, C08L 1/02;**
**A61L 24/043, C08L 89/00**

## Description

### Technical field

The present invention is related to a biodegradable sheet-shaped adhesive/reinforcement for tissues, which is able to adhere to living tissues so as to reinforce and cover adhered part of the living tissues, and may be used as a hemostatic material, an air leak preventive material, an adhesion preventive material, a suture reinforcement, an adhesive sheet, or the like. In particular, the present invention relates to a sheet-shaped tissue adhesive/reinforcement, in which a two-reactant adhesive powder is fixed to a biodegradable porous base.

### Background technology

Typical commercially available sheet-shaped tissue adhesive/reinforcement include: fibrinogen formulations; formulation using polysaccharides; and formulation using aliphatic polymers. "Tacosil" ™ (registered trademark) (CSL Behring Co., Ltd.) as a fibrinogen formulation is a sheet of collagen sponge, to which blood-derived fibrinogen, thrombin, etc. are attached. For such attaching, it seems that a treatment such as spraying a solution of fibrinogen and thrombin on a collagen sponge and then drying is performed (Patent Documents 2 to 3). On the other hand, as a commercially available formulation using a porous sheet of a polysaccharides or an aliphatic polymer, which does not contain an adhesive component, there have been known "Surge Cell" ™ (Johnson End Johnson Co., Ltd.: fabric sheet of cellulose oxide) and " "Neoveil" ™ (Gunze Co., Ltd.: non-woven fabric of polyglycolic acid). In addition, commercially available is an absorbable local hemostatic material (trade name "Integra" ™ manufactured by Koken Co., Ltd., sold by Nippon Zoki Pharmaceutical Co., Ltd.), which is made by spinning atelocollagen derived from calf dermis into fibers shaped as cotton fibers, and is chemically cross-linked with a polyepoxy compound.

As a tissue adhesive or tissue reinforcement that does not use a biological raw material, there is a two-reactant one composed of a mixed powder of aldehyded glucan and modified poly-L-lysine according to the proposal of the present applicant (Patent Document 1). This tissue adhesive or tissue reinforcement is sprayed on a to-be applied area, as it is as a powder, then gelled by being sprayed with a physiological saline solution, and adheres to the applied area in a form of a sheet. Animal tests have already confirmed the hemostatic effect and the air leak prevention effect on the lung resection surface (for example, Non-Patent Document 1 below).

### Prior art documents

#### Patent documents

Patent Document 1: WO2008/066182A (JP4571693B)
Patent Document 2: JP1986(S61)-034830B
Patent Document 3: WO 2012/029971A
Patent Document 4: JP2017-192560A

#### Non-patent document

Non-Patent Document 1: Development of new biodegradable hydrogel glue for preventing alveolar air leakage, J. Thoracic and Cardiovascular Surgery, 134 (5): 1241-8 (2007 Nov).

### Outline of the invention

### Problems to be solved by the invention

However, since fibrinogen formulations such as "Tacosil" are made from blood components, there is a risk of blood-derived infectious diseases. The "Surge cell", which does not use blood components as a raw material, utilizes the original blood coagulation ability of the patient, to which it is applied. Blood is absorbed into the porous sheet of the "Surge cell" to form blood clots, which realize a tissue reinforcing effect. After sticking to an area for blood stanching, it is necessary to hold for a few minutes until blood clots are formed. Meanwhile, with regard to "Neoveil", which is a porous sheet of aliphatic polymer, it is recommended to use fibrinogen formulations in combination with the "Neoveil" in order to reinforce its deficient adhesiveness to the biological tissue. Therefore, the conventional sheet-shaped tissue reinforcements would not have both of safety and convenience.

The present invention has been made to solve such a problem, and is intended to realize effects of adhering to and reinforcing of the tissues, with regard to sheet-shaped tissue adhesive/reinforcement, without using either fibrinogen or the blood coagulation ability derived from the patient's body.

Means to solve the problems

The present invention is defined by the independent claims. Advantageous embodiments are described in the dependent claims, the following description and the drawings.

The medical-use two-reactant adhesive disclosed in WO2008/066182 (Patent Document 1) is applied to an incision or a wound of a living tissue in a powder state, and then crosslinked or polymerized, at a time such as a physiological saline solution is added, so as to be converted to hydrogel state from powder state as a result of reacting of the two reactant components with each other. The hydrous gel by itself has adhesiveness to living tissues and has a characteristic of hindering leakage of liquids and gases. Already, animal tests have confirmed hemostatic effect and effect of preventing air leakage on the surface of lung resection, as the effects of tissue adhesion/reinforcement. The powder of this medical two-reactant adhesive is porous and has a random shape, which is obtained from an aqueous solution by drying such as freeze-drying and then pulverizing, and has an average particle size of 10 to 150 $\mu$m.

The inventors of the present invention have made various attempts to obtain a sheet-shaped tissue adhesive/reinforcement by using the above-mentioned medical two-reactant adhesive in the form of powder. However, this medical-use two-reactant adhesive does not dissolve in a solvent other than water. Further, when dissolved in water, the reaction between the aldehyde group of the aldehyded glucan and the amino group of the modified poly-L-lysine takes place; and with the hydrogel, in a state where the reaction is almost completed, it has decreased adhesiveness to the living tissue. Therefore, this adhesive would not be able to be attached to the porous base sheet by a method same as that for attaching fibrinogen, thrombin, and the like.

Even if the aldehyded glucan powder and the modified poly-L-lysine powder are separately dissolved in water, when aqueous solution of the aldehyded glucan is sprayed on the base sheet and dried, and then an aqueous solution of modified poly-L-lysine is sprayed thereon, for example; then the reaction between the aldehyde group of the aldehydeglucan and the amino groups of the modified poly-L-lysine proceeds. Although area-wise separately applying the two reactant solution in a manner of dots or stripes would be possible, it requires very complicated process and thus would not be realistic.

On the other hand, it is also conceivable to attach the powder of the above-mentioned medical two-reactant adhesive to a porous base by encapsulating in capsules or by using a binder. However, the use of capsules and binders may prevent the reactant components from rapidly coming into contact with water, and may cause delaying or incomplete curing (gelation). In addition, the process for adhering to the porous base may become complicated.

The inventors of the present invention have learned that such intended one is able to be realized by a simple method as below using absolute ethanol or the like, in course of diligent investigation to obtain a sheet-shaped tissue adhesive/reinforcement using the above-mentioned medical two-reactant adhesive---the powder of the medical two-reactant adhesive was dispersed in absolute ethanol; then added to a biodegradable porous sheet such as a collagen sponge sheet, by spraying or dipping; and then dried to remove ethanol. Then, surprisingly, the powder of the medical two-reactant adhesive was fixed to the biodegradable porous sheet, and even if the porous sheet was vibrated or slightly impacted, the medical two-reactant adhesive powder would not come off and fall off.

Further, the sheet-shaped tissue adhesive/reinforcement thus obtained maintained reactivity and adhesiveness in the same manner as the powder of the medical two-reactant adhesive. That is, when the incised portion or the wound site of the living tissue is appropriately sprayed with a physiological saline solution, the sheet-shaped tissue adhesive/reinforcement quickly absorbs water and is hardened (gelled) to be firmly adhered to the living tissue.

This cured water-containing gel also has adhesiveness to a biodegradable porous sheet (hereinafter, referred to as a "base sheet" when appropriate) having a hydrophilic group on its surface. When the adhesive/reinforcement is applied, it also has the effect of adhering the biological tissue to the base sheet. Therefore, the sheet-shaped tissue adhesive/reinforcement is not only able to bond the living tissues to each other, but also able to protect and reinforce a wound site of the living tissue when attached thereto. For example, if the tissue adhesive/reinforcement is attached to the wound site of a living organ, the wound site would be firmly covered and closed until healing is completed, and a reinforcing action would be performed to prevent deformation or a crevice formation due to an external force or the like.

That is, according to the sheet-shaped tissue adhesive/reinforcement of the present invention, tissue-reinforcing action of the base sheet is added onto to the tissue-reinforcing action and the covering/closing action of the cured water-containing gel itself. Resultantly, it is able to achieve further reinforcing of the tissues, as well as a strong sealing action that physically impedes the passage of liquids and gases. In particular, it is able to achieve compression hemostasis (astriction) at sites that are eruptive or exudative, and thus convenience is expanded. In the present patent application, "tissue adhesion/reinforcement" is used to include hemostasis, prevention of air leakage, prevention of tissue adhesion, reinforcement by sutures, and protection of the traumatic surface; and obtainable effects includes: blocking the passage of liquid and gas from both inside and outside the living tissue; preventing contact between the living tissue and other living tissue or solid matter; and retaining surfaces of the living tissues.

In the sheet-shaped tissue adhesive/reinforcement of the present invention, gelation of the two-reactant adhesive powder for realizing adhesion is started by adding water. The water that initiates gelation may be of any form or type. Examples of the water as the gelling initiator include: water on living tissues, which makes blood or body fluids; physiological saline, and glucose solution.

Advantages of the invention

The risk of infection would be eliminated or reduced, and it would be excellent in handleability and tissue reinforcement effect.

A brief description of the drawing

Fig.1 is a series of photographs showing a state (Example 2) when a two-reactant adhesive powder (LYDEX ™) was put into ethanol having various water contents and stirred.

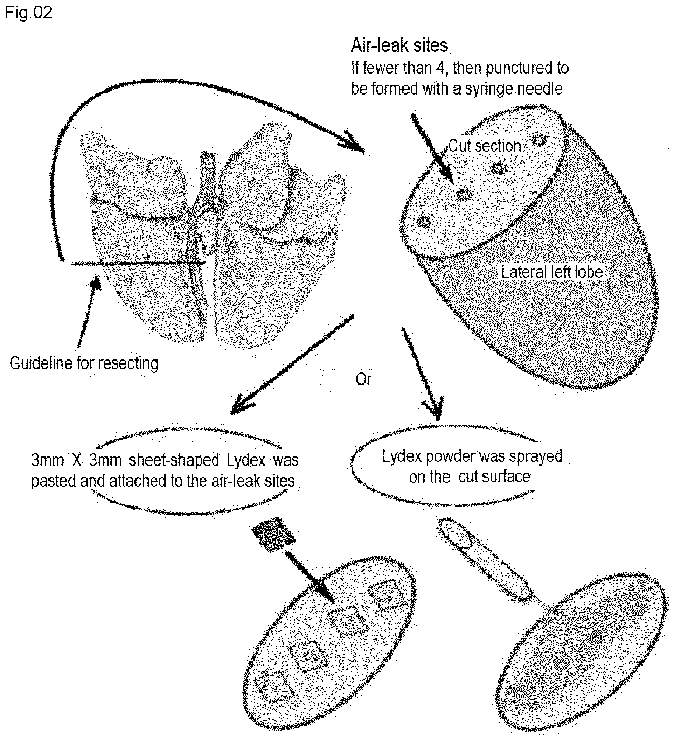

Fig.2 is a set of illustrations for showing the procedure of an air leak test (Example 3) using rabbit lungs.

Fig.3 is a set of photographs taken from a vertical diagonal direction, showing two types of mesh sheets (2.5 × 5 cm) obtained by spinning and knitting poly-L-lactic acid (PLLA), in a folded state.

Fig.4 is a set of photographs showing the state of forming an adhesive resin layer (Example 4-1) on one of the mesh sheets of Fig.3, having a finer stitch (mesh) ("PLLA mesh dense").

Fig.5 is a set of photographs showing the state of forming an adhesive resin layer (Example 4-2) on another of the mesh sheets of Fig.3, having a coarser stitch (mesh) ("PLLA mesh coarse").

Fig.6 is a schematic view showing the main points of an in vitro adhesive force evaluation test (Example 6) for the adhesive sheet of Example 4-1.

Fig.7 is a photograph showing an appearance of an adhesive strength evaluation test (Example 6).

Fig.8 is a graph showing the results of an adhesive strength evaluation test (Example 6).

Fig.9 is a right side view of a powder spray device (spray gun; JP2017-222915A) used for applying a two-reactant adhesive (LYDEX), where the right-side housing is omitted.

Fig.10 is a photograph collectively showing: an adhesive sheet (Example 7), in which a fibrous (as cotton fibers) collagen sheet is used as the base sheet; and an adhesive sheet (Example 8), in which, by using the base sheet same as above, hydroxypropyl cellulose (HPC) is added to the resin powder to be applied, to amount 20% of the resin powder.

Fig.11 is a photograph showing an appearance of the adhesive sheet of Example 7 when the sheet is once rolled into a scroll shape and then opened and stretched.

Fig.12 is a photograph of the adhesive sheet of Example 8, in otherwise same with FIG.11.

Fig.13 is a set of photographs showing appearances, at each stage of forming an adhesive sheet, of those of Reference Example 1 using absolute ethanol.

Fig.14 is a set of enlarged photographs, at two magnifications (× 100 and × 400 ), showing the "EtOH-saturated" and the "EtOH partially dried" shown in the middle rank ("4") of FIG13, as well as the surface after drying, which is shown in bottom part of Fig.13.

Fig.15 is an enlarged photograph of a cut surface of the dried sheet shown in bottom part of Fig.13.

Fig.16 is a set of photographs similar to Fig.13 showing appearances of those of Reference Example 2 using non-hydrous acetone, at respective stages.

Fig.17 is a set of enlarged photographs, at two magnifications (× 100 and × 400 ), showing the "Acetone-saturated" and the "Acetone partially dried" shown in the middle rank ("4") of Fig.16 as well as the surface after drying shown in the bottom part of Fig.16.

Fig.18 is a set of left-side and right-side photographs shown side by side, showing: the two-reactant adhesive powder (left side) after being dispersed in non-hydrous acetone and then completely dried; and the original two-reactant adhesive powder (right side).

Fig.19 is a set of photographs showing appearances of those of Reference Example 3 using 2% hydrous acetone, at respective stages----from the left, the dispersion liquid in a petri dish; an appearance immediately after applying this dispersion liquid to the base sheet; the adhesive sheet after drying; and an appearance when the adhesive sheet is picked up with tweezers and lifted up.

Fig.20 is a photomicrograph of the surface of the adhesive sheet after drying shown in Fig.19.

Fig.21 is a set of photographs showing appearances of those of Reference Example 4 using a non-reactive similar mixed powder, at respective stages.

Fig.22 is a set of photographs showing appearances of those of Reference Example 5 at respective stages, in which only modified polylysine (second reactant; SAPL) was used instead of the two-reactant adhesive.

Embodiments for carrying out the invention

The sheet-shaped tissue adhesive/reinforcement of the present invention (hereinafter, also appropriately referred to as "adhesive sheet") is formed by fixing a powder of a two-reactant adhesive for medical use onto a biodegradable continuous porous sheet (base sheet). At the time of this fixing, a third material such as an adhesive, a binder, or a covering material is not interposed. The fixing here means a state in which all or most of the powder of the two-reactant adhesive is adhered to the base sheet, at least to the extent that the powder would not fall off entirely or mostly even when the sheet-shaped tissue adhesive/reinforcement is curved as bowed. In a preferred embodiment, such fixing is presumed to be caused by dissolving of a portion of the two-reactant adhesive powder, particularly the surface layer of the modified poly-L-lysine powder, as described later in detail.

The two-reactant adhesive here is particularly in the form of a mixed powder as described in WO2008/066182 (Patent Document 1), and reacts in the presence of water so as to form a hydrogel. In detail, the two-reactant adhesive is a mixed powder comprising: a first reactant formed of a powder of aldehyded glycan (aldehyded polysaccharide, particularly water-soluble aldehyded polysaccharide) having a weight average molecular weight of 1,000 to 200,000; and a second reactant formed of a powder of acid anhydride-added (partially carboxylated) poly-L-lysine (B1) having a weight average molecular weight of 1,000 to 20,000, which is obtained by adding carboxylic acid anhydride to a certain poly-L-lysine (A); wherein the first and second reactant are mixed so that molar ratio of aldehyd group to amino group becomes 0.9 to 3.5; and the aldehyded glycan is derived from water-soluble or water-dispersible polysaccharide, such as glucan (a polymer of D-glucose) by oxidizing the polysaccharide with periodic acid or periodate to introduce 0.1-1.0 aldehyde groups per anhydrous glucose unit. The glycan here is an $\alpha$-glucan in a preferred embodiment, and is dextran or dextrin for example. The glycan (polysaccharide) may also be glucosaminoglycan such as hyaluronic acid, guar gum, locust bean gum, carrageenan, hydroxypropylmethyl cellulose, hydroxypropyl cellulosic or the like.

The powder of glycan aldehyde and the powder of acid anhydride-added poly-L-lysine (B1), before being fixed to the base sheet, are both porous material having a random shape (a shape far from a sphere) and having average particle size of 10 to 150 $\mu$m. Such randomly shaped porous materials are powders obtained respectively from an aqueous solution of glycan aldehyde and an aqueous solution of the acid anhydride-added poly-L-lysine, by drying such as freeze-drying and by subsequent pulverizing with a high-speed rotary blade crusher or the like; or the equivalent powder. By mixing the powders thus obtained, of the aldehyded glycan and the powder of the acid anhydride-added poly-L-lysine (B1), at a predetermined ratio, a mixed powder as a two-reactant adhesive is obtainable. The mixed powder is preferably stored in a closed container or the like so as to maintain the water content at 2.0% or less, particularly 1.0% or less.

The two-reactant adhesive preferably comprises: a first reactant consisting of a powder of aldehyded glycan having a weight average molecular weight of 1000 to 200,000; and a second reactant consisting of a powder of partially carboxylated poly-L-lysine, which is obtained by reacting a poly-L-lysine having a weight average molecular weight of 1000 to 20,000 with succinic anhydride or glutaric anhydride so that the residual amino group ratio becomes 70 to 93%; wherein the powders of the first and second reactant are mixed with each other so that a molar ratio of the aldehyde group to amino group is 0.9 to 2.0; and wherein the aldehyded glycan is obtained by oxidizing dextran or dextrin with periodic acid or periodate so that 0.2 to 0.5 aldehyde groups were introduced per anhydrous glucose unit (monosaccharide unit). Further, the water content is preferably 2.0% or less. The medical two-component reactive adhesive is designed to adjust and set a period until the hydrogel collapses in a range of 2 to 3 days to 4 weeks or more, by varying amount of aldehyde groups having been introduced in the aldehyded glycan in a range of 0.2 to 0.4 per anhydrous glucose unit (monosaccharide unit).

In a preferred embodiment, the average particle size of the aldehyde glycan powder and the acid anhydride-added poly-L-lysine powder gradually becomes a more preferable range as it progresses from (1) to (7) in the following range. (1) 1 to 500 $\mu$m, (2) 5 to 350 $\mu$m, (3) 10 to 250 $\mu$m, (4) 10 to 150 $\mu$m, (5) 15 to 120 $\mu$m, (6) 20 to 100 $\mu$m, (7) 20 to 80 $\mu$m. Although 10 to 150 $\mu$m is a particularly preferable range, 15 to 120 $\mu$m or the like is more preferable. Here, the average particle size may be obtained using an image analysis program (for example, the image analysis particle size distribution measurement software "MacView" of Mountech Co., Ltd. may be used) or the like, from the image obtained by the stereoscopic microscope; by finding biaxial average diameter (simple average x of major axis length and minor axis length) and length-wise averaging these diameters ($\Sigma x^2 / \Sigma x$). The average aspect ratio (major axis length / minor axis length) of the powder is, for example, 1.3 to 3.0, particularly 1.5 to 2.0. The average particle size, for example, may be measured more easily by a laser diffraction particle size distribution measuring device SALD-2200 (laser diffraction / scattering method, using batch cell) and analysis program (WingSALD II-2200 for Japanese software) manufactured by Shimadzu Corporation; and almost the same result as the above image analysis may be obtained.

The glycan aldehyde powder and the acid anhydride-added poly-L-lysine powder, which form a two-reactant adhesive, typically have a biaxial average diameter distribution in a manner that, 90% or more is included in a range of 10 to 150 $\mu$m while 80% or more is included in a range of 20 to 100 $\mu$m. In addition, the average particle size of the aldehyded glucan powder may be 1.1 to 2.0 times the average particle size of the acid anhydride-added poly-L-lysine powder.

The base sheet has small sizes of pores that piercing through the sheet in its thickness direction so that all or most of the powder of the above-mentioned two-reactant adhesive is not able to pass through. Preferably, the base sheet is disintegrated and is absorbed or excreted in the living body within the period same as that of the above-mentioned medical two-reactant adhesive, for example, within a period of 0.3 to 3 times, particularly 0.5 to 2 times, of that of the two-reactant adhesive. However, depending on the intended use, the base sheet may be disintegrated much earlier than the two-reactant adhesive. For example, due to regeneration of living tissue at the wound site, it is possible that the tissue needs to be adhered, but the reinforcement with the textile is not so necessary. On the other hand, even if the fibers and the like constituting the base sheet remain in the living tissue for a relatively long period of time, the function of the living tissue may not be adversely affected.

The base sheet may be a textile sheet such as a non-woven fabric, a woven fabric, a knitted fabric, or a mesh sheet, and may be a non-textile open-cell porous sheet, such as those in a form of a sponge (foam) having continuous pores, in a form of suede (composite of fine fibers) and in a form of a fine lattice or mesh, or honeycomb.

The thickness of the base sheet may range, for example, from 10 $\mu$m to 1000 $\mu$m (1 mm), from 15 $\mu$m to 500 $\mu$m (0.5 mm), and particularly from 20 $\mu$m to 300 $\mu$m. In particular, when the base sheet is a textile sheet formed of fibers having relatively high strength, its thickness may be 10 $\mu$m to 150 $\mu$m, particularly 20 $\mu$m to 100 $\mu$m or 20 $\mu$m to 50 $\mu$m.

The base sheet is, in a preferred embodiment, a mesh sheet or knitted fabric formed of biodegradable filaments, and is in particular that obtained by warp knitting, such as tricot and raschel, so as to reduce elasticity. The mesh sheet or knitted fabric may be a single-layer sheet and may also be a multi-layer stacked sheet of knitted fabrics, and might be a fabric obtained by double knitting, three-dimensional knitting, or the like.

When the mesh sheet is adopted, the diameter of the yarns for forming the mesh sheet, other than its nodes or junctions, may be, for example, 20 to 300 $\mu$m. Further, in consideration of required shape stability, flexibility and the like, either of multifilament yarns or monofilament yarns may be adopted as required. Diameter of the opening or aperture of such a mesh sheet may be, for example, 0.1 mm to 3 mm, particularly 0.2 mm to 2 mm. The aperture ratio of the mesh sheet (value obtained by dividing total area of the openings or apertures by whole area of the sheet) may be, for example, 50 to 95%, particularly 60 to 90%. With such a mesh sheet, it is able to support the adhesive resin layer and increase the reinforcing effect while minimizing the thickness and weight.

When the knitted sheet is adopted, the aperture ratio (value obtained by dividing the total area of stitch openings or apertures by whole area of the sheet) may be set to, for example, 20 to 50%. Further, knitting yarns forming the fabric may have a porosity of, for example, 30 to 80% by using multifilament yarns or staple yarns.

The base sheet is, in one preferred embodiment, a non-woven fabric or paper, which is formed of biodegradable fibers. In particular, filaments or staples made of a biodegradable polymer are used to produce the base sheet, by a needle punch method, a spunlace method, a spunbond method, a papermaking method or the like.

In one preferred embodiment, the base sheet is a textile sheet or a fine mesh or reticulated sheet, which has, at least in vicinity of the surface of the sheet, fine-fiber bundles or raised sites, piles, or fine uneven or relief structures so as to be beneficial for the base sheet to retain the powder of the medical two-reactant adhesive and to adhere to the powder.

In a preferred embodiment, the textile or non-textile porous sheet that forms the base sheet has relatively large pores when outer surface and/or cut surface is observed with an optical microscope. The diameter of such relatively large pores is 500 $\mu$m or less, 400 $\mu$m or less, or 300 $\mu$m or less. In a specific embodiment, the average pore diameter (median diameter; D50) is, for example, 20 to 150 $\mu$m for pores having a diameter of 1 $\mu$m or more as observed with an optical microscope. The porosity (volume ratio of pores) of the base sheet is, for example, 50 to 97% or 60 to 95%.

Examples of the biodegradable polymer forming the base sheet include proteins such as collagen, other amino acid polymers, polysaccharides or derivatives thereof, aliphatic polyesters such as aliphatic hydroxyl-carboxylate polymers, or derivatives thereof, and polyvinyl alcohol (PVA) or a derivative thereof. Here, the "derivative" is one obtained by imparting or adjusting properties such as biodegradability, by introducing a functional group, oxidizing, reducing, replacing an atom in the chemical formula, or the like. All of such biodegradable polymers have hydrophilicity and have good adhesiveness to the above-mentioned two-reactant adhesive.

Preferred biodegradable polymers include: collagen, dextran, dextrin, and aliphatic polyesters, and polydioxanone (PDS). Preferred aliphatic polyesters include: polyglycolic acid (PGA), glycolic acid/lactic acid copolymer (PLGA), polylactic acid (PLLA, PDLLA), and lactic acid/caprolactone copolymer. The biodegradable polymers listed here are preferable because they are easily designed to be disintegrated after a lapse of a predetermined period because of disintegrating from the inside due to hydrolysis or the like. In addition to the above, other lactic acid-based polymers and polydepsipeptides may also be preferably adopted. Also adoptable are polymers such as polycaprolactone (PCL), polyglyconate (copolymer of trimethylene carbonate and glycolide), cellulose oxide, chitin and its derivatives.

Specific examples of the biodegradable polymer include the following commercially available products provided by BMG Co., Ltd.

(1) Polyglycolic acid (PGA): Melt flow rate (MFR) at 240 °C and 10 kg load is 3.0 to 9.0 g /10 min.

(2) Poly-L-lactic acid (PLLA): Weight average molecular weight Mw by GPC-light scattering method is 200,000 to 280,000; and melting point by DSC method is 180 to 195 °C.

(3) Glycolic acid / DL-lactic acid copolymer (PGDLLA): The molar ratio of glycolic acid to lactic acid is 20:80 to 30:70, the weight average molecular weight Mw by the GPC-light scattering method is 220,000 to 280,000, and the DSC method. The melting point is 50-60 °C.

(4) L-lactic acid / ε-caprolactone copolymer (LCL)

- LCL (75:25): The molar ratio of L-lactic acid to ε-caprolactone is 70: 30-80: 20, the weight average molecular weight Mw by the GPC-light scattering method is 400,000 to 800,000, and the melting point by the DSC method is 150 to. 175 °C.
- LCL (50:50): The molar ratio of L-lactic acid to ε-caprolactone is 45:55 to 55:45, the weight average molecular weight Mw by the GPC-light scattering method is 200,000 to 600,000, and the melting point by the DSC method is 80 to 130 °C.

(5) Poly-p-dioxanone (PDO): The melting point by the DSC method is 100 to 110 °C. Melt flow rate (MFR) at 240 °C and 10 kg load is 3.0-9.0 g / 10 min.

In one preferred embodiment, the base sheet is: a foamed sheet having a communicating porous structure formed of a biodegradable resin such as polylactic acid or an aliphatic polyester; or a mesh sheet having a large number of vertically communicating pores due to a fine honeycomb structure.

Specific examples of the base sheet include: collagen sponge sheet ("single layer type" of "Pernac G Plus ™" of Gunze Co., Ltd.; press sheet of "INTEGRAN" of Koken Co., Ltd.), collagen fiber sheet (Nippi Co., Ltd.'s "Collagen Fiber Sheet"), polyglycolic acid non-woven fabric ("Neoveil" ™, Gunze Co., Ltd.), oxidized cellulose and oxidized regenerated cellulose fabric ("Surge Cell" and "Interseed" ™, Johnson & Johnson Co., Ltd.), non-woven fabric of chitin ("Vesquitin" ™, Nipro Co., Ltd.) and the like.

The collagen sponge sheet may be obtained, for example in accordance with disclosures of Example 3 of JP4681214B, by adjusting the collagen solution to acidic, cross-linking with glutaraldehyde, and then freeze-drying and heat-drying.

In a preferred embodiment, the powder of the above-mentioned two-reactant adhesive is fixed to at least one surface (one among front and back surfaces) of the base sheet to form a porous layer made of the two-reactant adhesive. The thickness of this porous layer may be adjusted by appropriately increasing or decreasing the amount of the two-reactant adhesive powder applied onto the base sheet. In addition, in present patent application, "fixing" includes not only the form adhered to or laminated on the surface of the base sheet, but also the form existing inside the base sheet.

The thickness of the resin layer (adhesive resin layer) by the two-reactant adhesive on the base sheet after the two-reactant adhesive is fixed to the base sheet and dried is, for example, 50 μm to 1000 μm (1 mm), 100 μm to 800 μm (0.8 mm), 150 μm to 600 μm (0.5 mm), and particularly 200 μm to 500 μm. When the thickness of the base sheet is, for example, 15 μm to 500 μm (0.5 mm) or 20 μm to 300 μm, the thickness of the adhesive resin layer may be 0.5 to 20 times, 0.8 to 10 times or 1 to 5 times, and so on, of the thickness of the base sheet. The thickness of such an adhesive resin layer corresponds to the amount of the powder of the two-reactant adhesive applied at a time the two-reactant adhesive is fixed to the base sheet.

The amount of the two-reactant adhesive applied (based on dry weight) at the time of manufacturing such a sheet-shaped adhesive/reinforcement (adhesive sheet) may be, for example, 30 to 500 mg / cm$^2$, 40 to 400 mg / cm$^2$, 50 to 300 mg / cm$^2$ or 60 to 250 mg / cm$^2$, and in particular 70 to 200 mg / cm$^2$, 80 to 200 mg / cm$^2$ or about 100 to 150 mg / cm$^2$. If the thickness of the porous layer by the reactant adhesive or the mass per unit area is excessively large, the flexibility of the sheet-shaped tissue adhesive/reinforcement is reduced, and if it is excessively small, the adhesive strength and sealing for leakage prevention may become insufficient. The thinner the porous layer of the two-reactant adhesive on the base sheet is, the more flexible it is, and therefore the better the sticking to the wound site is. However, in addition to the above-mentioned adhesive strength and sealing property, the operability of applying the two-reactor adhesive to the base sheet is varied by the applied amount of the two-reactant adhesive and nature and physical properties of the base sheet. Therefore, the coating amount of the two-reactant adhesive may be preferably adjusted in consideration of these variations comprehensively.

In a preferred embodiment, the sheet-shaped tissue adhesive/reinforcement (adhesive sheet) and the base sheet for this have a flat shape extending along one plane, so that the adhesive sheets of a predetermined size may be stacked, or may be wound in a roll shape. In otherwise, the adhesive sheet may be transformed to a curved shape in advance according to the shape of the wound site or the affected area to be applied. The adhesive sheet product may take a shape curved in only one direction to form an arc-shaped cross section, or a cup shape so as to form a part of a spherical surface. Depending on situations, the adhesive sheet product might take a shape of a thread, a rod, a bag, a pipe or the like.

In order to fix the powder of the two-reactant adhesive to the base sheet using ethanol, the water content of ethanol is less than 2%, 1.5 % or less, less than 1.5%, 1% or less or less than 1%; especially 0.9% or less, 0.8% or less, 0.7% or less, 0.6% or less, or 0.5% or less. The water content of ethanol is preferably set to, for example, 0.1% or more, 0.2% or more, 0.3% or more, or 0.4% or more. Thus, the water content of ethanol may be set in a range of: 0.3 to 1.5%, or 0.3 to 1%, particularly 0.3 to 0.8%. As the water content of ethanol becomes higher than the above upper limit values, it becomes more difficult to uniformly fix the mixed powder of the two-reactant adhesive (powder of aldehyde dextran, and powder of succinic anhydride) onto the base sheet. In particular, it becomes more difficult to disperse the mixed powder of the two-reactant adhesive, and the mixed powder may partly or entirely form a lump.

It is particularly advantageous to adopt ethanol as a solvent (dispersion medium) for fixing the two-reactant adhesive powder to the base sheet. This is because: by adopting low-water content ethanol having a water content of 0.2% to 1.5%, especially 0.2% to 1.0%,the mixed powder of the two-reactant adhesive are able to be satisfactorily fixed onto the base sheet. In addition, when ethanol is used, problems such as toxicity caused by a solvent that remains slightly after recovery / removal of the solvent and drying are eliminated or significantly reduced when to use the sheet-shaped tissue reinforcement for the human body.

When preparing a sheet-shaped tissue adhesive/reinforcement (adhesive sheet), a pharmaceutically acceptable additive may be added. In particular, when to fix and attach the powder of the two-reactant adhesive onto the base sheet, the additive may be dissolved or dispersed in advance in ethanol so as to be used in the preparing. Examples of the additive include amino acids, sugars, fatty acids, salts, sugar alcohols and surfactants. By appropriately combining one or more of these additives with a two-reactant adhesive, it is able to vary the solubility and flexibility of a hydrogel to be generated by absorbing water, as well as the base sheet, and to vary gelation rate, and adhesion strength between the adhesive sheet and the biological tissue, when using the sheet-shaped tissue adhesive/reinforcement.

Additives will adhere to the surface of the base sheet and the surface of the porous layer formed of the two-reactant adhesive, in course of evaporation of the ethanol from the state of being dissolved or dispersed in ethanol. It is also considered that the fixation of the two-reactant adhesive is caused by the partial dissolution of a part of the powder of the two-reactant adhesive, particularly by the partial dissolution of the surface layer portion of the modified poly-L-lysine powder. Therefore, it is considered that the additive is incorporated into the solidified portion after being once dissolved as in the above. At a time the sheet-shaped tissue adhesive/reinforcement is used, most of the additive is considered to be trapped inside the hydrogel formed by absorbing water.

Pharmacological components may be added in place of or in combination with the above additives. Examples of the pharmacological component include antibiotics, antiinflammatory agents, blood circulation improving agents, steroid agents, enzyme inhibitors, cytokines, vitamins, enzymes and the like. As a result of that the tissue adhesive/reinforcement contains a pharmacological ingredient, the tissue adhesive/reinforcement may also be used as a kind of drug delivery system intending sustained release of the pharmacological ingredient so that the pharmacological ingredient is gradually released from the hydrogel.

The application/fixing of the two-reactant adhesive onto the base sheet, for manufacturing the sheet-shaped tissue adhesive/reinforcement (adhesive sheet) is performed by, for example, any of (i) to (iv) below.

(i) Discharging dispersion liquid to the base sheet

The two reactant adhesive powder is forcibly dispersed in the above-mentioned low water content ethanol. Then, thus-obtained relatively unstable dispersion (suspension) is applied onto the base sheet by spraying, dripping or dropping, particularly from upward, or by discharging with other method.

For example, one part by weight of a powder of a two-reactant adhesive having an average particle size of 20 to 80 $\mu$m is added to 8 to 15 parts by weight of ethanol having a low water content; is stirred to be uniformly dispersed in the ethanol, by a small stirring mechanism; and discharged to be applied onto the base sheet while being stirred. For example, it is able to adopt a slit coater or a curtain coater provided with a stirring mechanism in the liquid pool portion, or a spray coater. As the spray coater, an air spray type may be adopted in which spraying is performed with a compressed gas, and for example, a spray gun with a paint agitator (for example, "Agitator spray gun" of Anest Iwata Co., Ltd.) is adoptable. An electrostatic spray or ultrasonic spray coater may also be used. In some cases, a syringe-dispenser may also be used.

For simplified experimentation, application may be made by using a dispenser such as a dispensing pipette, while stirring with a vibration motor or ultrasonic waves if appropriate or necessary. Adoptable stirring mechanisms here include a coin-shaped vibration motor used for mobile phones (for example, a $\varphi$11 mm DC vibration motor manufactured by Nippon Densan Copal Electronics Co., Ltd.); and a stick-shaped (pen-shaped) ultrasonic washer (for example, an equivalent to the UW A1 series of Sharp Corporation).

If a biodegradable polymer is added to ethanol as described later, the viscosity of the dispersion medium formed of ethanol may be increased to achieve thickening stabilization in a manner that the dispersion of the two-reactant adhesive powder is stably retained.

(ii) Abutting the base sheet against the dispersion liquid layer

The same dispersion liquid as in the above (i) is spread on a surface, and then, the base sheet is abutted against thus formed layer of the dispersion liquid so as to achieve the application. The surface on which the dispersion liquid is spread may be a flat surface or a curved surface such as a roller surface. In advance, a metal surface may be made to be non-adherent, if appropriate or necessary, by lining the metal surface with a silicone resin or a fluororesin.

In order to spread the dispersion liquid uniformly on the surface, the coater or the dispenser same as in the above (i) may be used. That is, a slit coater, a curtain coater, a spray coater or a dispenser, which has a stirring mechanism in the liquid pool, or the like may be used. A roll coater such as a direct gravure coater may also be adopted, by which the dispersion liquid is applied to the roller surface and then transferred to the base sheet.

In one embodiment, the same dispersion liquid as in the above (i) is placed on a non-adhering flat surface, and is spread so as to have a uniform thickness, and then, on which the base sheet is placed. For example, the dispersion liquid of the above (i) is uniformly applied onto a horizontal and flat metal plate lined with silicone resin or fluororesin, or onto a bottom surface of a vat or tray, by using a curtain coater or dispenser equipped with a stirring mechanism. Subsequently, the base sheet is placed on the layer of the dispersion liquid so as to cover the layer. Then, in such state, a process step of removing the ethanol is performed; and then, the base sheet and the adhesive resin layer formed on one surface of the base sheet are able be peeled off from the non-adhering surface formed by the lining. Here, the base sheet on which the adhesive resin layer is formed may be peeled off before drying of the adhesive sheet is completed. Further, for example, the base sheet may be fixed to the surface of the drum lined with a silicone resin, and then be sequentially abutted against the surface coated with the dispersion liquid of the above (i). Here, for example, it is conceivable to use a rubber roller as a coating roller and bring it into contact with a drum, on which the base sheet is attached.

(iii) Supplying the dispersion medium after applying the powder to the base sheet

Only the mixed powder of the two-reactant adhesive is applied to the base sheet by spraying or the like, and then ethanol is supplied onto the base sheet by spraying or dropping.

For such spraying ethanol, for example, an ordinary spray bottle may be used, for example. It is also adoptable a needle spray gun (Tomita Engineering Co., Ltd.), in which a fixed-quantity discharge valve mechanism and a spray gun are integrated. In addition, in order to apply ethanol into or soak with ethanol the base sheet, it is not limited to spraying or dropping, but for example, a sponge surface or sponge roller soaked with ethanol may be abutted against the base sheet. Further, it is also possible to saturate the base sheet with ethanol by placing the powder coated base sheet on a thinly spread layer of ethanol.

(iv) Powder-coating after supply of dispersion medium

Only the powder of the two-reactant adhesive is sprayed on the base sheet having been soaked with ethanol.

In order to soak the base sheet with ethanol; for example, the base sheet is immersed in ethanol, or the base sheet is uniformly saturated with ethanol by the above needle spray gun or spray coater.

When the two-reactant adhesive is applied and fixed to the base sheet as in (i) to (iv) above, particularly when the powder of the two-reactant adhesive as in the above (i) is dispersed in the ethanol and sprayed, a biodegradable polymer soluble in ethanol such as hydroxypropyl cellulose (HPC) and hydroxypropyl methyl cellulose (HPMC) may be added in advance to the ethanol. By adding, for example, 5 to 30% of such a biodegradable polymer relative to the weight of the two-reactant adhesive, flexibility and toughness may be imparted to the layer of the two-reactant adhesive in the sheet-shaped tissue adhesive/reinforcement after drying..

As the biodegradable polymer adopted here, any of such polymer may be used so long as it is uniformly dispersed even if the additional polymer is not completely dissolved in the dispersion medium. Further, it is not necessary to dissolve or disperse such polymer in advance of mixing with the mixed adhesive powder, the additional polymer in a form of powder may be mixed with the mixed adhesive powder before the dissolving or dispersing.

When the adhesive sheet is used for hemostasis, a blood coagulation promoter may be added by various methods similar to the addition of the biodegradable polymer described above so as to be contained in the adhesive resin layer or the like. Specifically, the adhesive sheet may be added by a dry preparation of a coagulation-inducing agent such as: thrombin, which exerts a hemostatic effect by a thromboplastin-like action; or hemocoagulase, which is an enzyme hemostatic agent made from snake venom. The amount of coagulation inducer such as thrombin added is, for example, 200 to 5000 units (IU) or 500 to 3000 units (IU) on a 100 mm × 50 mm base sheet, that is, 4 to 100 units (IU) or 10 to 60 units (IU) per square centimeter. In some cases, tranexamic acid, adrenaline, or a derivative thereof can be used as the blood coagulation promoter.

Various bio-derived polymers may be named as a preferable type of the biodegradable polymer, which is to be added to the adhesive sheet at a time the two-reactant adhesive is applied and fixed to the base sheet as described in (i) to (iv) above. As such bio-derived polymers, collagen and a collagen derivative such as gelatin are particularly preferable in preventing cracks in the adhesive resin layer. In this regard, according to circumstances, biodegradable peptides such as sericin, casein, fibrin and the like may be used; and the above-mentioned biodegradable polymers that are adoptable to form the base sheet may also be used alone or in combination with each other, or in combination with collagen or the like. Whereas these polymers may be added by various methods similar to the above-mentioned manners of addition of the biodegradable polymers, but in particular, the polymers may be added in form of powder having average particle size similar to that of the mixed powder of the two-reactant adhesive, so as to be mixed with the mixed powder of the two-reactant adhesive.

An adhesive obtained by adding a moisturizing ingredient or plasticizer such as glycerin may be added at a time of applying and fixing the two-reactant adhesive to the base sheet as described in (i) to (iv) above so as to impart flexibility to the resin layer, and so as to curb occurrence of cracks when being bent. Adding amount of the moisturizing ingredient may be, for example, 0.5 to 30% or 1 to 25%, particularly 2 to 15% or 2 to 10%, relative to weight of the two-reactant adhesive. Other than the glycerin, adoptable moisturizing ingredients include propylene glycol, 1,3-butylene glycol, 1,2-pentanediol, 1,2-hexanediol, polyethylene glycol, sorbitol, maltitol, and sodium dl-pyrrolidone carboxylate, sodium lactate, polyglycerin, sodium hyaluronate and trimethylglycine. These moisturizing ingredients may be added, for example, by being mixed with the mixed powder of the two-reactant adhesive.

In order to apply the powder of the two-reactant adhesive, in a state of being dispersed in ethanol or the like, on the base sheet or on a bottom surface of a vat or tray in a manner as in (i) to (ii) above, adoptable is a slit coater or a spray gun provided with a stirring mechanism in a liquid reservoir or the like. Further, at a time of such applying, a moving stage or a belt conveyor may also be used for relative movement between the liquid discharging part and the base sheet.

In order to apply the powder of the two-reactant adhesive on the base sheet at a time of fixing this powder on the base sheet by the above methods (iii) to (iv), the powder may be applied by a powder sprinkler that drops evenly from small holes or mesh openings in a manner of sieves, or by a powder discharge device in a manner of a spray gun or dispenser. In course of such, an electrostatic coating method or a flow immersion method as used for powder coating may also be used if necessary or appropriate. For example, applying of the powder may be made by providing, at the bottom of the powder storage tank, a powder discharge portion in which a large number of small holes are arranged in an elongated region, and by dropping the powder through the powder discharge portion while moving the base sheet in a direction crossing the elongated powder discharge portion. In course of such, a high voltage may be applied between such screen portion and table surface on which the base sheet is placed so that the powder of the two-reactant adhesive is uniformly sprayed, by a kind of electrostatic coating method. For example, a device having a mechanism such as "electrostatic screen dusting machine" of Berg Industries Co., Ltd. may be used. Further, for example, the powder may be applied by putting a base sheet in a fluidized state of the powder with dry compressed air (fluid immersion method). Furthermore, in the (iii) above, applying of the powder may be made by placing a lump of powder on the base sheet and then spreading it evenly using a rubber roller or rubber blade (rubber wiper).

In one preferred embodiment, by using a horizontal drive mechanism (X-axis drive mechanism) in the left-right direction having a servomotor or the like and a horizontal drive mechanism (Y-axis drive mechanism) in the front-rear direction having a servomotor or the like, the powder discharge portion and the base sheet may be continuously or sequentially moved with each other, in horizontal directions, in course of applying the powder of the adhesive on the base sheet. In other word, the powder discharge portion may be attached to the drive end of the XY-axis drive mechanism having the X-axis drive mechanism and the Y-axis drive mechanism, so as to form an XY-axis discharge portion-driving device (for example, "desktop coating plate coating machine" of NCC Co., Ltd.). In otherwise, the base sheet may be placed on an XY table provided with an XY axis drive mechanism, and the powder may be applied from a powder discharge portion having a fixed position.

In one preferred embodiment, the medical powder spray device of JP2016-063919A or the powder spray device of JP2017-222915A may be adopted. These powder spray devices are pistol-shaped spray guns, by which the powder is sprayed with compressed air, and which are configured to be lifted up and be turned on and off by one hand of the practitioner. In order to apply the powder of the two-reactant adhesive onto the base sheet using these powder spraying devices, the above-mentioned XY-axis discharge portion-driving device or the XY table, or in otherwise, a robot arm, or other moving stage or a belt conveyor may be used. In course of such, an electrostatic coating method or the like may also be used.

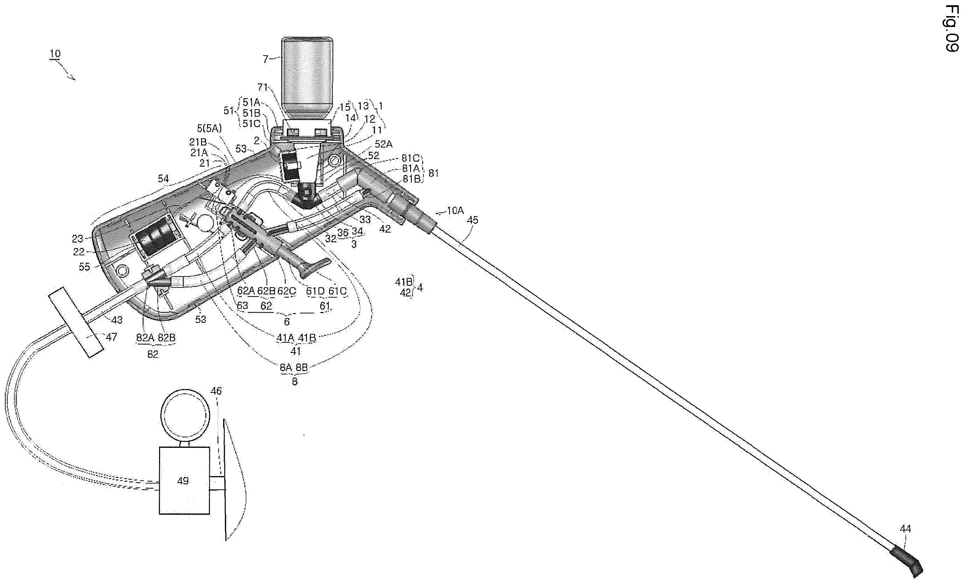

As shown in Fig.9, the powder spraying apparatus described in JP2017-222915A comprises: (i) a funnel member 1, on top part of which the powder container 7 is attachable, or with which the powder container 7 integrally provided; (ii) the first three-way joint 3, first opening 31 of which is connected to discharge port 11B at the lower end of the funnel member 1, as well as airflow supply pipe 41 and the delivery pipe 42, which are respectively connected to second and third openings 32, 33 of the first three-way joint 3 and a housing 5 accommodating hereto-mentioned members, (iii) a vibration motor 2 provided in the funnel member 1; (iv) a bypass airflow pipe 8, which branches out from the airflow supply pipe 41 and joins the delivery pipe 42 without passing through the first three-way joint 3, and (v) an On-Off mechanism, which is for switching between: a spray coating state (On-state), in which the compressed gas is sent through the coating flow path 4A passing through the airflow supply pipe 41, inside of the first three-way joint 3, and the delivery pipe 42 and also through the bypass airflow pipe 8; and a standby state (Off-state), in which the compressed gas is sent only through the bypass airflow pipe 8. The funnel member 1 and the first three-way joint 3 are attached to the housing 5 in a manner as swayable and swingable.

More generally, the powder spray device has: a storage portion capable of storing powder; a vibration mechanism capable of applying vibration to the storage portion from a time point before the start of spray coating; a delivery path for sending out the powder falling from the storage part to the nozzle, together with the compressed gas, at a time of the spray coating state (On-state); and a bypass path for sending out the compressed gas toward the nozzle without passing through a site, to which the powder falls from the storage portion, at a time of the standby state (Off-state). Such a powder spray device is suited for spray coating a powder, which has a random shape as described above, such as a two-reactant adhesive particularly used in the present patent application, and which easily absorbs moisture and thus causes blocking of the delivery path.

After applying the two-reactant adhesive to the base sheet as described in (i) to (iv) above, recovery of ethanol and drying may be made in a stationary state for example, by heating in a nitrogen atmosphere if necessary or appropriate. For example, "Tray shelf-powder drying system SDP-500" (Institute of Creative Chemistry Co., Ltd.) may be used. In otherwise, a vacuum dryer may be used, which is equipped with a fixed-temperature heating mechanism.

After drying, thus obtained sheet-shaped tissue adhesive/reinforcement (adhesive sheet) may be sterilized by: ultraviolet rays (UV), electron beam, ethylene oxide gas (EOG) or $\gamma$-ray. In order to avoid moisture absorption and the like, the adhesive sheet may be stored in a desiccator, or as sealed in a bag formed of a laminated film having an aluminum layer or the like.

In the above coating / fixing method, it has been described that a mixed powder of a two-reactant adhesive is used. It is also possible to: spray and apply L-lysine (SAPL) powder, then spray absolute ethanol evenly; subsequently, on top of this, spray and apply aldehyded dextran (AD) powder, and then spray again absolute ethanol.

The sheet-shaped tissue adhesive/reinforcement (adhesive sheet) obtained as above comprises: a base sheet having a biodegradable and porous structure; and an adhesive resin layer, which is fixed and formed on the base sheet. This adhesive resin layer contains a first reactant comprising an aldehyded glycan and a second reactant comprising partially carboxylated polylysine, and has a granular structure (grains) derived from the powder of the first reactant and a connecting layer derived from the second reactant. By this connecting layer, the granular structure (grains) of the first reactant is connected to each other and fixed to the base sheet.

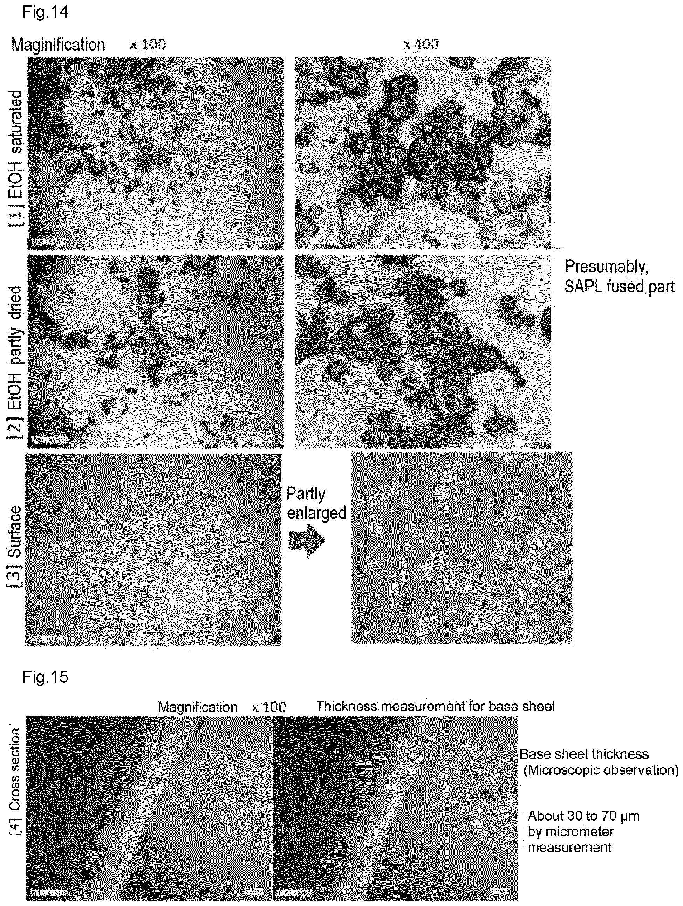

As shown in photographs on top part of Fig.14 for Reference Example 1, in a course of producing the adhesive sheet, It is considered that: the second reactant (partially carboxylated polylysine) absorbs the dispersion medium such as absolute ethanol and swells to form a paste, which is sticky and has some fluidity, and thus connects the powder of the first reactant (aldehyded glycan). Moreover, the second reactant or the paste, at the same time, is considered to stick to the base sheet and partially infiltrates into the large number of open-cell pores of the base sheet. After such infiltrating and subsequent drying procedure for removing the dispersion medium, the adhesive resin layer formed on the base sheet is presumed to adhere to the base sheet mainly through anchoring effect (mechanical bonding) due to portions filtrated into the pores. On the other hand, the powder of the first reactant (glycan aldehyde) is considered to mostly maintain their original shapes even in the dispersion medium so as to form a granular structure (grains) in the adhesive resin layer.

Examples

Hereinafter, embodiments of the present invention will be described by way of examples, but these do not limit the scope of the present invention.

<Example 1> Preparation of an adhesive sheet by applying a dispersion liquid to a cotton-like fibrous collagen sheet

0.5 g of the mixed powder of the two reactant adhesive was added into 6 mL (4.9 g) of "absolute" ethanol (ethanol 99.5 vol% or more, Wako pure chemical Co. 1st grade) in a glass container (10 mL vial), and then was uniformly dispersed by ultrasonic stirring after the container was closed by a stopper. Collagen sponge sheets were used as the base sheets. Specifically, Koken Co., Ltd.'s "Integran"-"Press Sheet" (100 mm × 50 mm, 0.2 g cotton-like fibrous collagen sheet) were used as they were as purchased.

A web page of "Integra" of Koken Co., Ltd. states as follows. "Integran is an absorbent topical hemostatic material formed by spinning a highly purified calf dermis-derived atelocollagen (collagen, which was solubilized by proteolytic enzymes and from which major antigenic expression sites have been removed) into fibers shaped as cotton fibers and by treating them with a polyepoxy compound for chemical cross-linking.."

After evenly dispersing as described above, the stopper of the glass container was opened. Then, while continuing ultrasonic stirring as needed or appropriate, the dispersion liquid in the glass container was sequentially sucked out by a dispensing pipette (Pipetteman P000 of MS Equipment Co., Ltd., equipped with a 1 mL tip) and applied to the entire surface of the base sheet. The application was carried out by dropping evenly on the above-mentioned 5 cm × 5 cm base sheet. Subsequently, the sheet was dried by leaving it in the air at room temperature overnight, then by putting it in a vacuum dryer and by vacuuming at room temperature so that ethanol was completely removed and overall moisture content became 0.2% or less. In this way, the thickness of the adhesive resin layer having irregularities was set to about 300 to 350 μm (0.3 to 0.35 mm). Then, the amount of the adhesive resin per square centimeter was considered to be 15 to 20 mg, excluding the peripheral portion of the base sheet. This amount was almost equal to or slightly less than the lower limit of the optimum one-time application amount of 20 to 30 mg / cm$^2$ when the powder of the two-reactant adhesive was used as it was.

While the adhesive sheet ("LYDEX sheet / collagen base") thus obtained was slightly stiff, the adhesive sheet may be made into a scroll with a diameter of about 2 cm by rolling it. At a time of such rolling, the adhesive resin did not fall off and the adhesive resin layer did not crack. When the surface and cut section of the adhesive sheet were observed with a high-performance stereomicroscope ("Digital Microscope VHX-5000" system manufactured by KEYENCE Co., Ltd.), the mixed powder of the two-reactant adhesive had minute protrusion structures, which are presumed to be derived from the mixed powder and especially from the powder of the aldehyded dextran, as densely formed over the entire surface.

The mixed powder of the two-reactant adhesive here was a mixed adhesive powder (average particle size of 80 μm) as a mixture of a powder of: aldehyded dextran, in which introduced amount of aldehyde group per glucose unit is 0.28; and a powder of succinic anhydride-added polylysine, in which a remaining ratio of free amino group is 89.5%; as being mixed at a weight ratio of 4/1. Manufacturing method of this is as described in WO2008 / 066182 (Patent Document 1). Thus, the mixed powder was obtained as in 1) to 3) below.

1) Preparation of powdered aldehyded dextran (first reactant; AD)

400 g of dextran (Meito Sangyo Co., Ltd., "Dextran 70") having a molecular weight of 70,000 was dissolved in 1600 mL of ion-exchanged water; and 50 g of sodium periodate (molecular weight 213.89) was dissolved in 800 mL of ion-exchanged water and then added to the solution of the dextran, and stirred in a water bath at 50 °C for 3 hours so as to proceed reaction. Then, the solution after the reaction was dialyzed, filtered through a 0.45 μm filter, and dried. Further, a pulverization was carried out using a small crusher (Wonder Crush Mill D3V-10, Osaka Chemical Co., Ltd.) to obtain a powdery aldehyded dextran (2.5 / 20). Here, (2.5/20) indicates the charging amount ratio of the sodium periodate to the dextran 70 (sodium periodate / dextran 70) forming the aldehyded dextran. The particle size of the powder was evaluated by using a stereomicroscope; and the average particle size was found as 90 μm. Furthermore, surface texture of the powder was observed with an electron microscope and was resultantly found to be a porous body. The average aspect ratio (ratio of the major axis to the minor axis) was about 1.6.

The amount of aldehyde group introduced per amount of sugar residue (moL) in the obtained aldehyded dextran was 0.28. The amount of aldehyde group introduced was measured by the redox titration method. Specifically, 20 mL of a 0.05 moL / L iodine aqueous solution, 10 mL of a 10 mg / mL aldehyded dextran aqueous solution and 20 mL of a 1 moL / L sodium hydroxide aqueous solution were placed in a 100 mL Meyer flask and stirred at 25 °C for 15 minutes. Then, 15 mL of a 6 v / v% sulfuric acid aqueous solution was added, and titration was performed with a 0.1 moL / L sodium thiosulfate aqueous solution. The end point was determined at a time point the reaction system became colorless and transparent; and, as the indicator, an aqueous starch solution was adopted.

2) Preparation of succinic anhydride-treated polylysine (second reactant; SAPL)

10 g of succinic anhydride (Nacalai Tesque) was added to 400 g of a 25 wt% ε-polylysine aqueous solution (molecular weight 4,000, Chisso Co., Ltd.), and the mixture was allowed to be reacted at 50 °C for 1 hour. The solution after the reaction was filtered through a 0.45 μm filter and dried. Further, a pulverization was carried out using a small crusher (Wonder Crush Mill D3V-10, Osaka Chemical Co., Ltd.) to obtain a powdery succinic anhydride-treated polylysine. Regarding the obtained succinic anhydride-treated polylysine, the remaining ratio of free amino groups (side chains and terminal amino groups that are not involved in the formation of peptide bonds) was determined and found to be 89.5%. For this measurement, the obtained succinic anhydride-treated polylysine was dissolved in water and added with a ninhydrin solution and a buffer solution of acetic acid / sodium acetate having a pH of 5.5; then, the mixture was heated in a boiling water bath for 3 minutes, and then rapidly cooled to give a sample solution. This was subjected to a test according to the ultraviolet-visible absorbance measurement method of the Japanese Pharmacopoeia, by which the absorbance at a wavelength of 570 nm was measured so that the amino group content in the sample solution was determined. The obtained powdered succinic anhydride-treated polylysine was evaluated using a stereomicroscope in the same manner as in the above-mentioned aldehyded dextran, and found to be a porous body having almost the same random shape. The average particle size was 80 $\mu$m. The average aspect ratio was about 1.7.

Regarding the obtained succinic anhydride-treated polylysine, the remaining ratio of free amino groups (side chains and terminal amino groups that are not involved in the formation of peptide bonds) was determined and found to be 84.7%. For this measurement, the obtained succinic anhydride-treated polylysine was dissolved in water and added with a ninhydrin solution and a buffer solution of acetic acid / sodium acetate having a pH of 5.5; then, the mixture was heated in a boiling water bath for 3 minutes, and then rapidly cooled to give a sample solution. This was subjected to a test according to the ultraviolet-visible absorbance measurement method of the Japanese Pharmacopoeia, by which the absorbance at a wavelength of 570 nm was measured so that the amino group content in the sample solution was determined.

3) Mixed adhesive powder

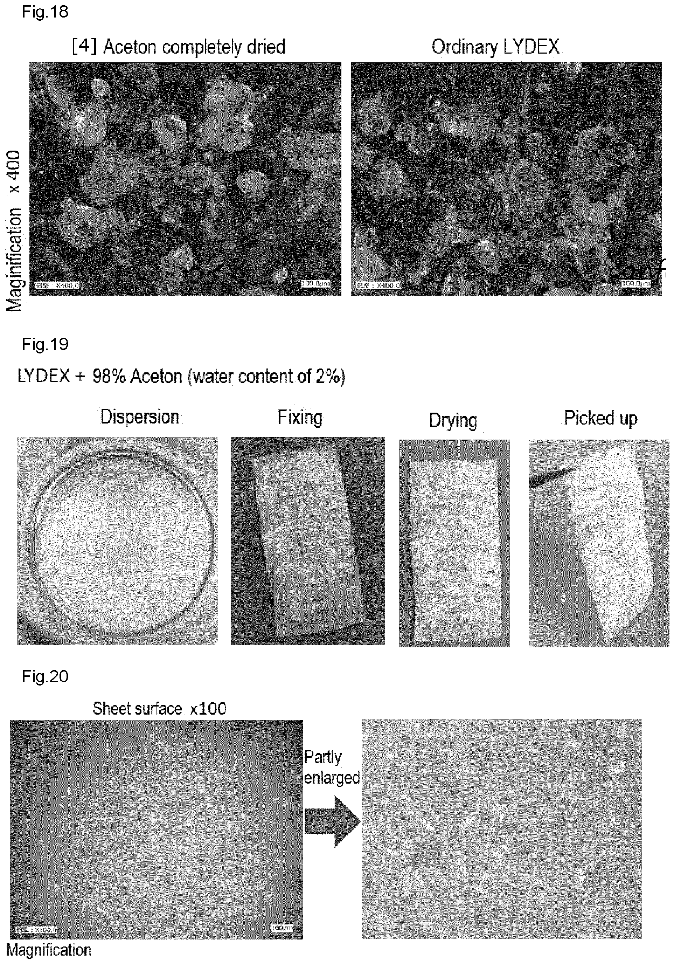

The above powdered aldehyded dextran and powdered succinic anhydride-treated polylysine were mixed at a weight ratio of 4/1 so that the mixed adhesive powder (average particle size of 80 $\mu$m) has an aldehyde group-to-amino group molar ratio at approximately 1. The obtained mixed adhesive powder is shown (in Fig.18). The bulk density of the mixed adhesive powder was 420 mg / cm$^3$. The mixed adhesive powder was hermetically stored so as to maintain the water content at 0.5 to 1.0%. In the present patent application, this mixed adhesive powder is also appropriately referred to as "Lydex ™". Unless otherwise specified, the above reaction charge ratio and mixing ratio were used. That is, succinic anhydride-treated polylysine used was obtained by reacting 100 g of $\varepsilon$-polylysine with 10 g of succinic anhydride; and the charged amount weight ratio of sodium periodate to dextran 70 at a time of introducing aldehyde group to the dextran was 2.5 to 20 (2.5 / 20). In the adopted mixed powder of a two-reactant adhesive ("LYDEX2.5 / 20"), such aldehyded dextran (AD) and such succinic anhydride-treated polylysine (SAPL) were mixed at a weight ratio of 4/1.

<Example 2> Effect of water content of dispersion medium

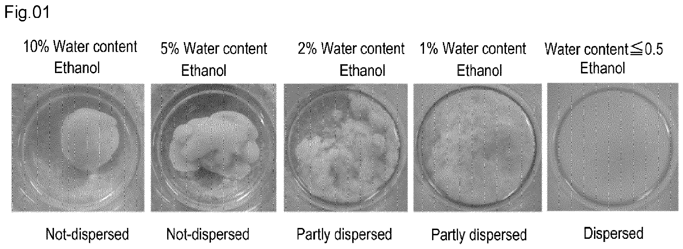

The mixed powder of the above two-reactant adhesive was dispersed in ethanol having different water contents, and the state of dispersion was observed. Dispersion media having a water content of 5 stages were prepared, each of which is 6 mL of ethanol, and which have water content levels adjusted to 0.5% or less, 1%, 2%, 5%, and 10%, respectively. Then, in the above glass container (10 mL vial), 0.5 g of the mixed powder of the two-reactant adhesive same as used in Example 1 was added to the ethanol and stirred with ultrasonic waves in a manner as in Example 1. Subsequently, obtained dispersion was put into a glass petri dish ($\varphi$32 $\times$ 20 mm) and was observed. Appearances of the dispersions are collectively shown in a set of photographs in FIG.1.

For the "0.5% or less", commercially available absolute ethanol (ethanol 99.5 vol% or more) was used as it was. After this experiment, it was confirmed that the water content in the absolute ethanol product was about 0.3-0.5%. Further, the hydrous ethanol having a water content of 1% to 10% is obtained by adding the calculated amount of water on the assumption that the water content of commercially available absolute ethanol is 0.4%.

As shown in Fig.1, when the water content is 5% or more, the phase containing the powder of the two-reactant adhesive was aggregated; and thus, it was not able to spread the adhesive powder throughout the dispersion medium or hydrous ethanol as a whole. When the water content was 2%, the adhesive powder was just possible to be spread throughout the entire dispersion medium, but was immediately undergone phase separation and thus was difficult to be dispersed uniformly. When the water content was 1%, the adhesive powder was possible to be dispersed almost uniformly as shown in Fig. 1; but, after being left for about 10 seconds, exhibited partial phase separation. On contrary, when "absolute ethanol" having a water content of 0.3 to 0.5% was used, no phase separation was observed by naked-eye observation until about 10 seconds even if the dispersion was left to stand after stirring.

From the results of Fig.1, it was known that the water content of ethanol presumably needs to be less than 2% or 1% or less, preferably 1% or less, and more preferably around 0.5%. The mechanism and cause of the great influence of water content were further investigated by experiments as mentioned later.

<Example 3> Evaluation of hemostatic effect

Hemostasis effect for incised tissue was evaluated by using an adhesive sheet ("LYDEX sheet / collagen base"), the base sheet of which was a cotton-like fibrous collagen sheet ("Integra" and "press sheet" 100 mm × 50 mm from Koken Co., Ltd.).

<Example 3-1 > Adhesive sheet of Example 1

Using the adhesive sheet obtained in Example 1, the hemostatic effect was evaluated as follows.
A median incision was made in the upper abdomen of a Japanese white male rabbit to expose the liver. The lateral left lobe of the liver was partially resected to a length of about 4 cm, and hemostasis was performed by lightly pressing with gauze whose weight was measured in advance. In this way, the amount of bleeding before application was determined and recorded, and eruptive bleeding was confirmed. After that, the hemostatic sheet obtained in Example 1 ("LYDEX sheet / collagen base") and the commercially available hemostatic sheet "Tacoseal" (registered trademark, CSL Behring Co., Ltd.) were used for hemostatic treatment.
Hemostasis treatment was performed by lightly pressing the hemostatic sheet against the incision and adhering it in the same manner as compression hemostasis. However, at the time of hemostasis treatment with the hemostatic sheet of Example 1, a small amount of physiological saline was sprayed if needed or as needed. That is, if and when a portion of the two-reactant adhesive is found as remained as a powder and as not in a form of the hydrogel, a physiological saline solution was sprayed to complete the gelation.
The amount of bleeding was calculated by sucking the leaked blood with gauze and by using the following formula as from the difference in weight before and after application. The results are shown in Tables 1 and 2 below.

Hemostasis ratio (%) = (before application-after application) / before application x 100

<Table 1> Adhesive sheet of Example 1

|  | Bleeding amount (g) |  | Hemostasis ratio (%) |
|---|---|---|---|
| Test No. | Before the treatment | After the treatment |  |
| 1 | 4.709 | 0.571 | 87.87 |
| 2 | 0.785 | 0.218 | 72.23 |
| 3 | 0.516 | 0.122 | 76.36 |
| 4 | 0.654 | 0.280 | 57.19 |
| 5 | 0.975 | 0.948 | 2.77 |
| Average |  | 0.428 | 59.58 |

<Table 2> Commercially available hemostatic sheet ("Taco Seal")

|  | Bleeding amount (g) |  | Hemostasis ratio (%) |
|---|---|---|---|
| Trial No. | Before the treatment | After the treatment |  |
| 1 | 2.471 | 0.388 | 84.30 |
| 2 | 3.821 | 1.901 | 50.25 |
| 3 | 1.827 | 0.568 | 68.91 |
| 4 | 1.187 | 0.742 | 37.49 |
| 5 | 2.313 | 0.916 | 60.40 |
| Average |  | 0.903 | 60.27 |

As is known from the results of Tables 1 and 2 above, no significant difference in the average bleeding was observed between: those using the adhesive sheet of Example 1 of the present application ("LYDEX sheet / collagen base sheet"); and those using a typical commercially available hemostatic sheet. Nevertheless, in the "Trials No.5" of Example 1, the hemostasis ratio became extremely low. This was presumably because extremely thin portions were formed in the adhesive resin layer at a time the hemostatic sheet or the adhesive sheet was manually prepared. Thus, excellent hemostasis performance would be achieved if manufacturing process for the adhesive sheet is improved by using a slit coater equipped with an agitation mechanism, or if defective ones of the adhesive sheets, which have thin portions of the adhesive layer of the like, are rejected by inspection procedure. In otherwise, the trial of lowest value may be omitted by considering presumable errors at a time of hemostasis procedure; and then the adhesive sheet of Example 1 would be superior to the commercially available hemostasis sheet.

<Example 3-2> Adhesive sheet by applying powder dispenser

Onto the base sheet same as in Example 1 ("Integran"-"press sheet" 100 mm $\times$ 50 mm of Koken Co., Ltd.), 6 mL (4.9 g) of "anhydrous" ethanol (ethanol 99.5 vol% or more, Wako 1st grade) was evenly applied to the entire surface using a spray-dispenser. Immediately after this, 0.5 g of the mixed adhesive powder ("LYDEX2.5 / 20") as in Example 1 was uniformly applied to the entire surface of the base sheet. Subsequently, the sheet was dried in a manner as in Example 1 to obtain an adhesive sheet.
For the application of ethanol and the mixed powder, a coating device (NCC Co., Ltd.'s "desktop coating machine") equipped with a spray-dispenser attached to the drive end of the XY axis drive mechanism was used. That is, the spray-dispenser was sequentially moved in the front-rear direction while swinging in the left-right direction in a manner of scanning, so as to achieve uniform application throughout entire surface of the base sheet.
The hemostasis ratio was evaluated by the method and procedure as in Example 3-1 above; and resultantly, a hemostasis ratio of 90% or more was obtained.

<Example 3-3> Thrombin-containing adhesive sheet

To 0.5 g of the mixed powder ("LYDEX2.5 / 20") of the two-reactant adhesive as used in Example 1, a dry thrombin powder containing 2000 IU of thrombin was added and uniformly mixed. Then, an adhesive sheet was prepared in exactly the same manner as in Example 3-2 above, except that the thrombin was added.
The hemostasis ratio was evaluated by the method and procedure as in Examples 3-1 to 3-2 above, and resultantly, a hemostasis ratio of 95% or more was obtained.

<Example 4> Adhesive sheet using a mesh sheet as a base sheet

Two kinds of mesh sheets were adopted as the base sheet, which were manufactured (spun and knitted) by Gunze Co., Ltd. by using poly-L-lactic acid (PLLA) of BMG Co., Ltd. The poly-L-lactic acid (PLLA) has weight average molecular weight Mw of 200,000 to 280,000 by GPC-light scattering method and has melting point of 180 to 195 °C by DSC method.

<Example 4-1 >

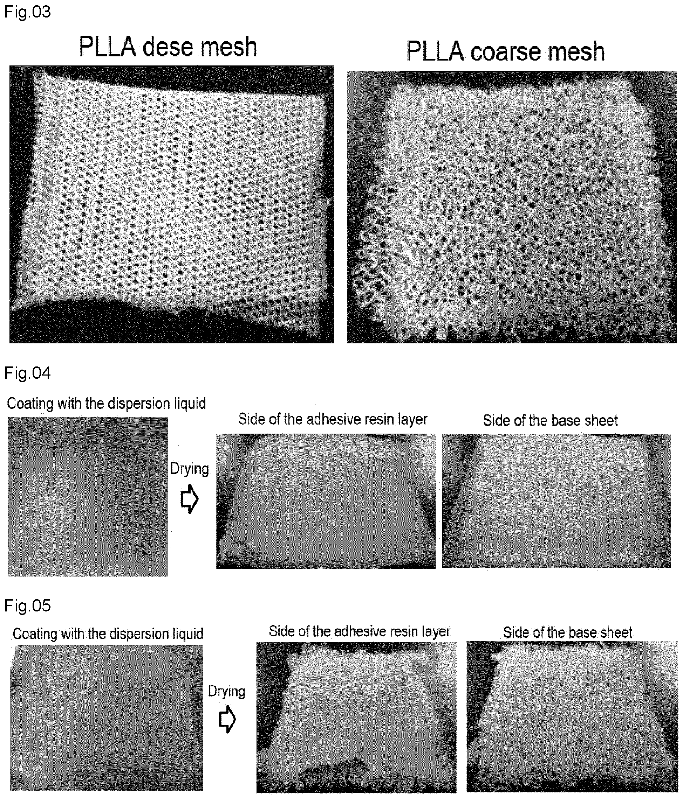

The first-kind mesh sheet ("PLLA dense mesh") is a single-layer knitted fabric, which is formed by tricot knitting (warp knitting, Denby knitting) using multifilament yarn (167dtx X 24), in which diameter of each opening (mesh opening) by the stitch is about 0.8 mm. The second-kind mesh sheet ("PLLA coarse mesh") is a single-layer knitted fabric, which is formed by tricot knitting (warp knitting, Denby knitting) using multifilament yarn (280dtx X 12), in which diameter of each opening by stitching is about 1.5 to 2 mm.
Using the above-mentioned first-kind mesh sheet ("PLLA dense mesh"), an adhesive sheet ("LYDEX sheet / PLLA mesh dense") was prepared by the procedure indicated in a set of photographs in FIG.4. Firstly, the above first-kind and second-kind mesh sheets were cut into 2.5 $\times$ 5 cm, folded in half, and the front side portion and the back side portion were fused to each other at the left and right edges by an ultrasonic sealer. On the other hand, the dispersion liquid of the two-reactant type adhesive as same as used in Example 1 ("LYDEX 2.5 / 20" 0.5 g + absolute ethanol 6 mL) was prepared. Immediately after the dispersion was prepared, the mixture was transferred from a 10 mL vial onto the bottom surface of a horizontally placed fluororesin petri dish (PTFE petri dish; inner diameter 50 mm) so as to be applied evenly and spread with a uniform thickness. Subsequently, the double-folded mesh sheet was put on the layer of the dispersion liquid placed on the bottom surface of the petri dish. Then, in exactly the same manner as in Example 1, vacuum drying was performed at room temperature so that the overall moisture content became 0.2% or less.
As a result, an adhesive sheet ("LYDEX sheet / PLLA mesh dense") was obtained as shown in the two photographs on the right half of Fig. 4. The rightmost photograph of Fig. 4 shows: the adhesive resin forming surface (upper side during immersion / drying); and the second photograph from the right in Fig. 4 shows the surface on the base side (lower side during immersion / drying). As shown in these photographs, the adhesive resin layer may also be formed on one side of the sheet also by a method of overlaying the base sheet on a spread layer of the dispersion liquid (i.e. "(ii) Abutting the base sheet against the layer of the dispersion liquid" as in above). The thickness of the adhesive resin layer was achieved to be about 300 to 350 $\mu$m (0.3 to 0.35 mm) as in Example 1.

However, the surface (back surface) on the base side also has tackiness in the presence of moisture. This is presumed to be because entire circumference of each yarn portion of the mesh sheet would have been coated with the adhesive resin, as is known from the right-most photograph of FIG.4.

<Example 4-2>

In a similar way, the mesh sheet with the coarser mesh opening ("PLLA coarse mesh") was adopted; and, the adhesive sheet- was manufactured as a trial in exactly the same way with the above. Appearances with regard to this is shown in Fig. 5, which is a set of photographs in same manner with those in Fig. 4. As shown in Fig. 5, it was able to form the adhesive resin layer on one-side surface, or lower side at a time of immersion and drying, of the mesh sheet as in the case of Fig. 4 above. However, the thickness of the adhesive resin layer was relatively non-uniform; and dot-shaped thin portions were formed. If the coating method and the like are improved, a usable adhesive sheet would presumably be obtainable as in Example 4-1 above.

<Example 4-3>

On the other hand, using polyglycolic acid (PGA) of BMG Co., Ltd. (melt flow rate (MFR) at 240 °C under a load of 10 kg is 3.0 to 9.0 g / 10 min), a third-kind of mesh sheet ("PGA mesh sheet") was manufactured (spun and knitted) and provided by Gunze Co., Ltd. in exactly the same manner as the first-kind mesh sheet ("PLLA dense mesh"). Using this, an adhesive sheet ("LYDEX sheet / PGA mesh sheet") was produced in exactly the same manner as the above "PLLA dense mesh". The properties of the mesh sheet formed of polyglycolic acid (PGA) were considered to be exactly same with that formed of poly-L-lactic acid (PLLA).

<Example 5> Sealant effect

Using the adhesive sheets obtained in Examples 1 and 4-3, the sealant effect on air leakage was evaluated by the following procedures (1) to (4). As comparative examples, the four types of sealants shown in Table 3 below and the section "(3) Application of sealant" were used.

(1) Preparation of experimental animals

To rabbits (Japanese white male rabbits), general anesthesia and thoracotomy were performed, and breathing was maintained by an artificial respiration device while securing an airway by a special mouthpiece. Breathing rate was set at 15 times / minute while inspiratory pressure was set at 10 cm $H_2O$.

(2) Preparation of air leak model

Posterior lobe of the left lung was partially excised and, was declamped to raise the inspiratory pressure to 30 cm $H_2O$. Then, physiological saline was dropped on the cut surface of the lung to confirm air leaks. When number of the air leaks was less than 4, the cut surface of the posterior lobe was pricked with an injection needle (18G) so that total number of the air leaks became 4. In this way, an air leak model was created.

(3) Application of sealant

Adopted sealants to stop the air leaks were; the adhesive sheet of Example 1 ("LYDEX sheet / collagen sponge"), and the adhesive sheet of Example 4-3 ("LYDEX sheet / PGA mesh") of the present patent application; and those of comparative examples. Each of these sealants was applied to the cut surface (affected part) by the following procedure.

- Adhesive sheets of Examples 1 and 4-3 of the present invention (A-1 and A-2 in Table 3)

The adhesive sheet was cut into 3 mm square pieces, applied to sites of the air leaks, and lightly pressed to be bonded with them. As in the above confirmation of the hemostasis effect, if some portions of the two-reactant adhesive would be remained as the powder per se and not become hydrogel, a physiological saline solution was sprayed on them to induce hydro-gelation.

- Powder of two reactant adhesive (LYDEX) (B-1 and B-2 in Table 3)

The two-reactant adhesive (LYDEX) powder was spray-applied to the cut surface, and a physiological saline solution was added dropwise as needed, to completely convert the powder into the hydrogel and to leave none of the adhesive in powder form. For this spray coating, the powder spray device (Fig. 9 of the present application) disclosed in JP 2017-222915A was used. Further, the coating amount (mass per area) of the adhesive resin was set to 25 mg / cm$^2$, which is slightly larger than that of the above-mentioned adhesive sheets of Examples 1 and 4-3.

Here, as the reactant adhesive (LYDEX), adopted in Test No. B-1 was the above-mentioned "LYDEX 2.5 / 20", which was used in all other examples of the present application while adopted in Test No. B-2 was "LYDEX 4.0 / 20". The "LYDEX 4.0 / 20" means that charging ratio (sodium periodate / dextran 70) to form the aldehyded dextran was set to "4.0 / 20" instead of "2.5 / 20", and that weight ratio of the aldehyded dextran to the succinic anhydride-treated polylysine was set in a manner that molar ratio of aldehyde group to amino group became approximately 1.

- Fibrin glue (B-3 in Table 3)

"Bolheel Tissue Adhesion Use" of KM Biologics Co., Ltd.'s was used according to an attached instruction, and thus mixture of Liquid A and Liquid B was applied to the cut surface or affected site through an attached dual syringe ("Bolheel Spray Set Type: End Spray").

- PGA mesh sheet + fibrin glue combined (B-4 in Table 3)

The polyglycolic acid (PGA) mesh sheet ("PGA mesh") used in Example 4-3 above was shredded into 5 mm squares. Then, using the "Bolheel preparer set; syringe set for rubbing-wise spray", the Liquid A was applied to the cut surface, then the shredded PGA mesh sheets were pasted onto the air leak sites, and then mixture of the Liquids A and B was applied to the cut surface.

(4) Air leak test by pressurization

The pressure was gradually increased up to 50 cmH$_2$O, and the presence or absence of air leakage was determined. Once air leakage was observed, then no further pressurization was performed, and the pressure at that time was taken as the air leakage-occurring inspiratory pressure.

The test results of the sealant effect are summarized in Table 3 below.

<Table 3> Sealant effect on air leaks

| Test No. | Sealant | Number of animals | Number of animals per inspiratory pressure that caused air leakage at | | | | Number of animals that did not cause air leakage at 50cm H$_2$O |
|---|---|---|---|---|---|---|---|
| | | | 20cm H$_2$O | 30cm H$_2$O | 40cm H$_2$O | 50cm H$_2$O | |
| A-1 | Adhesive sheet of Example 1 (Collagen sponge) | 5 | 0 | 0 | 3 | 2 | 0 |
| A-2 | Adhesive sheet of Example 4-3 (PGA mesh) | 5 | 0 | 1 | 2 | 2 | 0 |
| B-1 | Two-reactant adhesive powder (2.5/20) | 5 | 0 | 1 | 3 | 0 | 1 |
| B-2 | Two-reactant adhesive powder (4.0/20) | 5 | 0 | 2 | 3 | 0 | 0 |
| B-3 | Fibrin glue | 5 | 1 | 3 | 1 | 0 | 0 |
| B-4 | Fibrin glue+PGA mesh sheet | 5 | 0 | 2 | 2 | 1 | 0 |

As is known from the results in Table 3 above, the adhesive sheet of the invention ("sheet-shaped LYDEX"), or the sheet-shaped tissue adhesive/reinforcement exhibited almost the same sealant effect irrelevant to different kinds of the base sheet. Compared with the test results of the comparative examples carried out before, in which the two-reactant adhesive was used in the form of powder ("LYDEX (powder)"), and fibrin glue was used as it was or together with the mesh sheet, a higher sealant effect was observed by the adhesive sheet of the invention.

<Example 6> Adhesion strength evaluation test

The adhesive sheet ("LYDEX sheet / PLLA dense mesh") of Example 4-1 was used in vitro to evaluate the adhesive strength. The tensile strength test was carried out with reference to the JIS K 6850 adhesive---tensile shear adhesive strength test method for rigid adherends, by appropriately modifying their test pieces and test conditions.

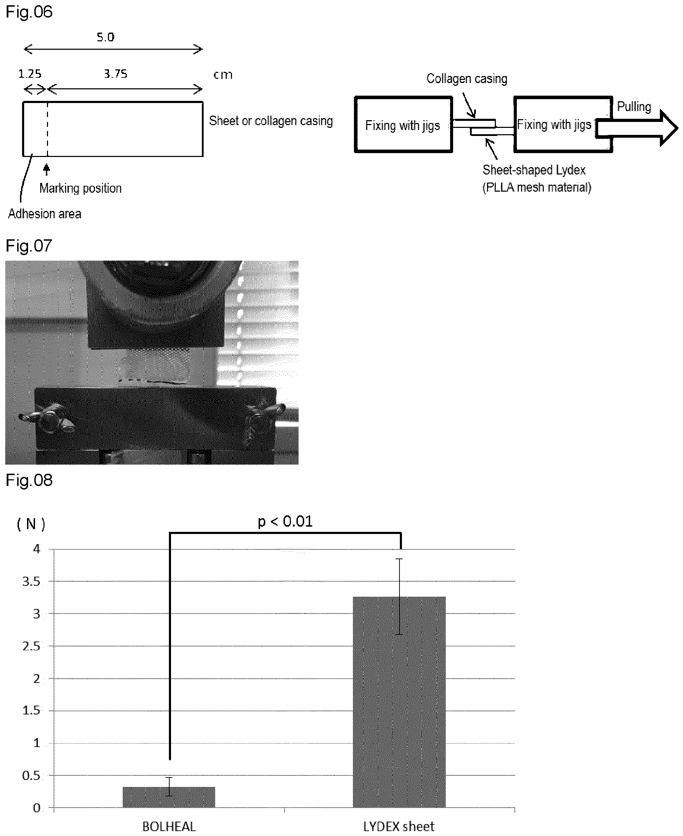

For the object to which the adhesive sheet is attached, a vertically cut open "collagen casing" of Nippi Co., Ltd., which is shaped as a tube having thickness of 28 μm and diameter of 20 mm, was used as a substitute for organs or the like. As shown in the schematic view of Fig.6, the adhesive sheet of Example 4-1 and the "collagen casing" are cut into small pieces of 5 × 2 cm, and an end portion extending from one end by 1.25 cm, on front or back side, was set as adhesion area, and marked with a magic pen. A small piece of the adhesive sheet and a small piece of the "collagen casing" were adhered to each other. At this occasion, 0.2 to 0.5 mL of physiological saline was sprayed on the adhesion area of the small piece of the "collagen casing", and then the adhesion area of the adhesive sheet of Example 4-1 was overlaid and pressed to be attached. Then, after the gelation time of 3 min (including the compression time of 30 seconds) has elapsed, almost all of the small pieces of the adhesive sheet and the small pieces of the "collagen casing" other than the adhesion area are clamped by the upper and lower chucks of the testing machine. In the tensile strength test, the test speed (crosshead climbing speed) was 50 mm / min, the load range was 10N 1%, and the recording speed was 200 mm / min.

The specific tensile strength test procedures were as follows.

1. The adhesion area (2.5 × 5 cm) and its vicinity of the small piece (2.5 × 5 cm) of "collagen casing" are wiped with a paper wiper for scientific experiments (Kimberly-Clark Corporation's "Kimwipe S-200"), which has been soaked with ethanol, and were allowed to stand for 30 seconds so as to be dried. After that, marking was made on the boundary between the adhesion area and the other area.
2. The adhesion area of the small piece of "collagen casing" was sprayed with physiological saline.
3. An adhesive sheet (LYDEX sheet) was put on the above adhesion area, and after spraying physiological saline, a weight was placed and pressed. Used for this was a weight (having about 200 g mass), which is formed by putting a vial containing water and on a PTFE petri dish (inner diameter 50 mm).
4. The weight was removed 30 seconds after starting the compression with the weight. Then, test piece was allowed to stand for another 2 minutes and 30 seconds. Therefore, the total gelation time was 3 minutes.
5. After the gelation time of 3 minutes had elapsed, the test piece was attached to the universal testing machine (tensile / compression testing machine) by clamping the vicinity of the marking with the chuck of the universal testing machine. For this, the adhesive sheet was clamped by the upper chuck while a small piece of the collagen casing was clamped by the lower chuck.
6. Starting the test (ascending the crosshead at 50 mm / min).

Further, as a comparative example, a small piece of "collagen casing" and a small piece of the mesh sheet ("PLLA mesh dense") used in Example 4-1 were adhered by using fibrin glue ("bolheel tissue adhesion-use" of KM Biologics Co., Ltd.). The dimensions of the small pieces to be bonded and the dimensions of the adhesion area were the same as in the above-mentioned example using the adhesive sheet. Further, the specific tensile strength test procedures were modified as below only on procedures "2" to "3" of the tensile strength test procedures "1" to "6"for the Example.

2. Approximately 0.3 mL of fibrin glue (for "bolheel tissue adhesion") was applied to the adhesion area of a small piece of "collagen casing" using the attached dual syringe.
3. Quickly after such application, to-be adhered area of the small piece of mesh sheet ("PLLA mesh dense") was attached to the adhesion area , and pressed by putting the above weight (having about 200 g mass).

The results obtained by the adhesive strength evaluation test are summarized in Tables 4 to 5 and Fig. 8 below.

<Table 4> Adhesive sheet of Example 4-1 ("LYDEX sheet / PLLA mesh dense")

| Adhesive sheet Specimen No. | Tensile strength (N) | Tensile strength Average | Standard deviation | Coefficient of variation of Tensile strength | Adhesive resin amount (g) |
|---|---|---|---|---|---|
| 1 | 3.790 | | | | 0.23 |
| 2 | 3.725 | | | | 0.26 |
| 3 | 2.435 | | | | 0.21 |
| 4 | 3.290 | 3.456 | 0.794 | 22.970 | 0.21 |
| 5 | 3.925 | | | | 0.21 |
| 6 | 2.795 | | | | 0.21 |
| 7 | 3.580 | | | | 0.20 |
| 8 | 2.575 | | | | 0.20 |

<Table 5> Fibrin glue + "PLLA mesh dense"

| Fibrin glue adhesion test piece No. | Tensile strength (N) | Average of Tensile strength | Standard deviation | Coefficient of variation of Tensile strength |
|---|---|---|---|---|
| 1 | 0.455 | | | |
| 2 | 0.120 | | | |
| 3 | 0.300 | | | |
| 4 | 0.210 | 0.329 | 0.140 | 42.655 |
| 5 | 0.270 | | | |
| 6 | 0.440 | | | |
| 7 | 0.545 | | | |
| 8 | 0.295 | | | |

The adhesive strength between the adhesive sheet of Example 4-1 ("LYDEX sheet / PLLA mesh dense") and the collagen casing was considerably large, and was considered to be sufficient for the purpose of adhering and reinforcing living tissues. After tearing off by the tension, the hydrogel as an adhesive layer also adhered and remained on the collagen casing. That is, it was considered that the adhesive layer was destroyed.

On the other hand, when the fibrin glue and the mesh sheet of poly-L-lactic acid (PLLA) were used, the hydrogel as an adhesive layer was present only on the side of the mesh sheet after peeling by pulling, and not remained as observed on the surface of the collagen casing. That is, the adhesive layer was not broken, and only the interface with the collagen casing was broken. It was considered that even if fibrin glue is simply used in combination with a biodegradable mesh sheet, it may not be so suitable as an adhesive sheet for adhering biological tissues.

<Example 7> Preparation of an adhesive sheet by spraying powder onto a cotton-like fibrous collagen sheet

As the base sheet, "Integra" and "Press Sheet" (100 mm × 50 mm, 0.2 g of cotton-like fibrous collagen) of Koken Co., Ltd. were used as they were, in a manner as in Example 1.

The mixed powder of the two-reactant adhesive ("LYDEX2.5 / 20") as same as used in Examples 1 and 4 was used. 1.0 g of the mixed powder of the two-reactant adhesive was evenly sprayed on an entire of base sheet (5 × 10 cm) using the powder spray device (spray gun) shown in FIG.9. A small amount (about 5%) of the sprayed mixed powder was dissipated and did not rest on the base sheet. After the application of the mixed resin powder, mist-form absolute ethanol was sprayed on the mixed powder using a small spray for drug administration. Then, the sheet was dried overnight at room temperature, and further dried in a vacuum dryer at room temperature for 6 hours.

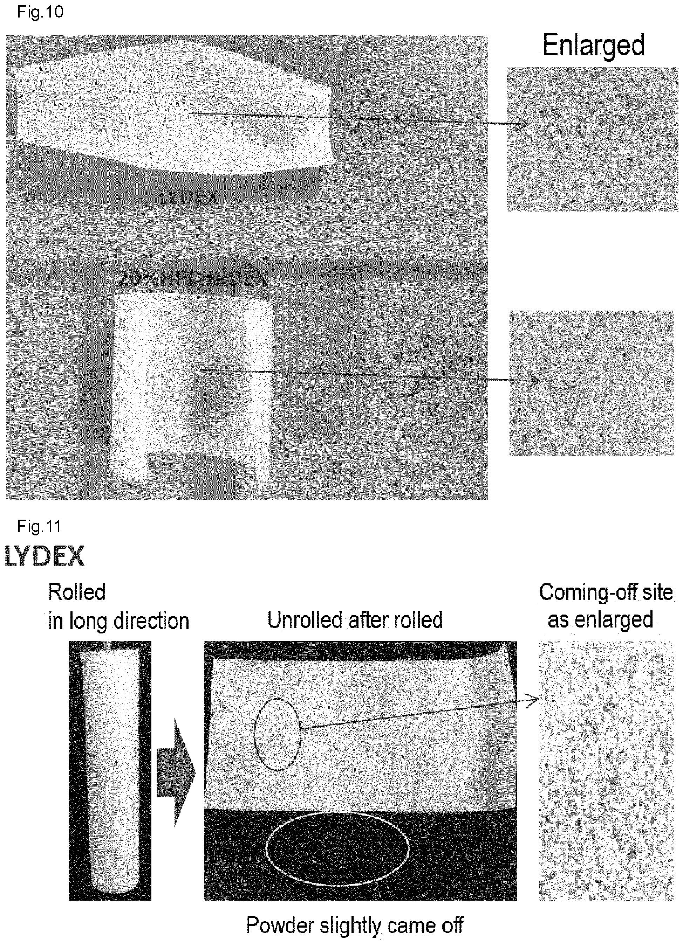

The adhesive sheet thus obtained was flat-shaped to some extent, before being putted while being in the dryer whereas, when the sheet was taken out from the vacuum state, it rapidly absorbed moisture, and severely warped. This state is shown in the upper part of the photograph of Fig. 10. When the warped adhesive sheet was placed in a dry box, it returned to a flat state. When it was tried to wound this adhesive sheet in a direction along the long side in a scroll shape so as to have a diameter of about 1 cm, it was able to be wound although it made a creaking noise. The states of being rolled and spread (opened and stretched) in this way is shown in the photograph of FIG.11. As shown in Fig. 11, the mixed powder of the two-reactant adhesive was slightly peeled off and fell off.

<Example 8> Addition of hydroxypropyl cellulose (HPC)

By experimentation as in Example 7, hydroxypropyl cellulose (HPC) was added as investigated when forming the adhesive resin layer composed of the two-reactant adhesive, so as to impart flexibility or toughness to the adhesive resin layer..

For this purpose, instead of 1.0 g of the mixed powder ("LYDEX2.5 / 20") of the two-reactant adhesive, 0.8 g of the mixed powder and 0.2 g of hydroxypropyl cellulose (HPC) were used as uniformly mixed using an ultrasonic stirrer. Such mixed resin powder was applied to the above base sheet (5 × 10 cm). Other conditions and procedures for producing the adhesive sheet are exactly the same as in Example 7. The hydroxypropyl cellulose used here is NISSO HPC SSL (D $_{50}$: 20 $\mu$m, 2% aqueous solution viscosity: 2-2.9 mPas) of Nippon Soda Co., Ltd., in which content of hydroxy-propoxy group (-OCsHsOH: 75.09) is 53.4-80.5%.

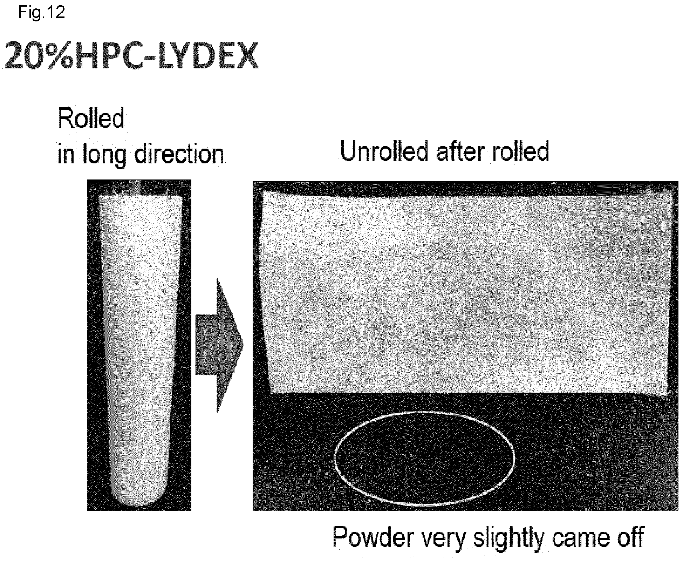

The obtained adhesive sheet is shown at the bottom part of the photograph in FIG. 10. Further, in exactly the same manner as in Example 7-1, the adhesive sheet was rolled into a scroll and then opened and stretched as shown in the photograph of FIG.12. As will be known from Fig.12, the mixed powder of the two-reactant adhesive was slightly peeled off. Compared with the results of Example 7-1 shown in Fig. 11, it is considered that the addition of hydroxypropyl cellulose (HPC) significantly reduced the dropout of the mixed powder of the two-reactant adhesive. That is, it is considered that the addition of hydroxypropyl cellulose (HPC) could impart flexibility or toughness to the adhesive resin layer.

The adhesive sheets obtained in Examples 7 and 8 were cut with scissors and their cross sections were observed with a high-performance stereomicroscope ("Digital Microscope VHX-5000" system manufactured by KEYENCE Co., Ltd.) so as to measure thickness of the adhesive resin layers. In detail, six locations of the adhesive sheet were randomly selected from the screen of the microscope device so as to measure the thickness. The results are shown in Table 6.

<Table 6> Thickness of adhesive resin layer (mm)

|  | Lydex | 20%HPC Lydex |
|---|---|---|
| 1 | 0.356 | 0.302 |
| 2 | 0.353 | 0.362 |
| 3 | 0.332 | 0.317 |
| 4 | 0.331 | 0.371 |
| 5 | 0.291 | 0.344 |
| 6 | 0.277 | 0.324 |
| Average | 0.323 | 0.337 |

As shown in Table 6, it was able to form the adhesive resin layer having a thickness of approximately 0.30 to 0.35 mm (about 300 to 350 $\mu$m). When the coating liquid added with hydroxypropyl cellulose (HPC) by 20% was used, it seemed that small-thickness portions become smaller in number and the overall thickness was slightly increased.

<Reference example> Examination of conditions and mechanism for forming the adhesive resin layer

In the following series of experiments (Reference Examples 1 to 8); in order to facilitate the experiment, instead of the biodegradable base sheet, a paper wiper for scientific experiments (Nippon Paper Crecia Co., Ltd.'s "Kim Wipe S" -200 ") was cut into 5 × 5 cm or 2.5 × 5 cm (reference example 3 only) and used as it was. The thickness of this paper wiper sheet was measured at a plurality of locations with a micrometer screw gauge and found to be about 30 to 70 $\mu$m. In addition, this paper wiper sheet ("Kimwipe S-200") is a durable sheet made of only bleached chemical pulp derived from wood (coniferous and hardwood), with less fluffing and paper dust, and is creped or textured. The pulp here is a mixture in a predetermined ratio, of: softwood-derived cellulose fibers having a length of 3 to 5 mm and a width of about 20 $\mu$m; and hardwood-derived cellulose fibers having a length of 1 to 2 mm and a width of about 50 $\mu$m.

<Reference example 1> Absolute ethanol

0.5 g of a mixed powder ("LYDEX2.5 / 20") of the two-reactant adhesive (LYDEX) used in Example 1 and the like was dispersed in 6 mL (4.9 g) of "anhydrous" ethanol (ethanol 99.5 vol% or more, Wako 1st grade), in exactly the same procedure as in Example 1. Then, this dispersion was applied to a sheet of paper wiper (5 × 5 cm of "Kimwipe S-200") in exactly the same manner and dried.

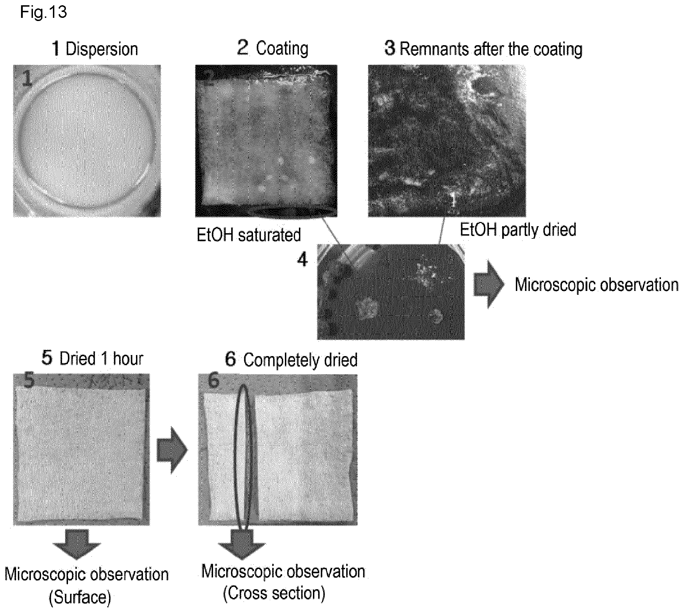

It was observed how the mixed powder of the two-reactant adhesive (LYDEX) changed into a resin layer with a high-performance stereomicroscope ("Digital Microscope VHX-5000" system manufactured by KEYENCE Co., Ltd.). A set of photographs in Fig.13 shows a series of stages from the preparation of the dispersion liquid to the sheet after the formation of the resin layer --- (1) the dispersion liquid in the petri dish; (2) the coated sheet immediately after being applied with the dispersion liquid to the sheet on a flat surface lined with silicone resin; (3) the coating layer (enlarged photograph) at the time of the drying starts locally; (4) the coating layers, which have spilled to nearby of the base sheet and transferred to another petri dish, at the time of (2) above, as well as a portion in which the drying started part at the time of (3); (5) the coated sheet after being left in the air at room temperature for about 1 hour; and (6) the coated sheet at the time completely dried. After leaving it to dry for about 1 hour, the coated sheet was transferred onto a brown paper wiper (Nippon Paper Crecia Co., Ltd.'s "Kimtowel 4 ply"); and the photographs of (5) and (6) show the dried coated sheet placed on the paper wiper.

Meanwhile, a set of photographs in Fig.14 shows planar images taken by a microscope at three of the stages at different magnifications (100 times and 400 times). Further, Fig.15 shows a cross section (100 times) of the coated sheet after complete drying.

Namely, Fig.14 shows the following [1] to [3] in order from the top; and Fig.15 shows the following [4].

[1] The two-reactant adhesive (LYDEX) soaked with the absolute ethanol. This was the coating layer before drying in (2) above, and was observed in a glass petri dish as in (4) above.

[2] A partially dried product, which was formed by being partially dried from the dispersion liquid having been spilled out from the sheet during application. The partially dried product had been peeled off from the flat surface on which the sheet was placed at the time of the above (3) and was observed.

[3] The surface of the sheet having the adhesive resin layer having been dried for about 1 hour, in the above (5).

[4] A cross section or a face formed by cutting with scissors, the completely dried sheet having the adhesive resin layer, in the above (6).

As shown in [1] of Fig.14, especially in the enlarged part within the right-side enlarged photo (× 400), observed were parts considered to be formed by fusing of succinic anhydride-modified poly-lysine (SAPL). And, as shown in [2] of Fig.14, especially in the right-side enlarged photo (× 400), the powders were bonded to each other at locally dried parts. Further, as is known from [3] of Fig.14 and [4] of Fig.15, after drying, the powders of the two-reactant adhesives were bonded to each other to form one adhesive resin layer. In this adhesive resin layer, each powder of random shape of aldehyde dextran (AD) formed a convex portion and was adhered on a paper wiper sheet which is a base sheet. According to the cross-sectional photograph of Fig. 15, the thickness of the paper wiper sheet was 39 $\mu$m at the thinnest part and 53 $\mu$m at the thickest part.

<Reference example 2> Non-hydrous acetone

0.5 g of the mixed powder ("LYDEX2.5 / 20") of the two-reactant adhesive (LYDEX) used in Example 1 and so on, was dispersed in 6 mL (4.7 g) of acetone (acetone 99.5vol% or more, Wako 1st grade) in exactly the same procedure as in the above Example 1 and Reference Example 1. Then, this dispersion was applied to a sheet of paper wiper (5 × 5 cm of "Kimwipe S-200") in exactly the same manner and dried. The acetone was used immediately after opening the reagent bottle, and it has been confirmed by the Karl Fischer titration method (chemical solution for ketones) that the water content was about 0.2 to 0.3%.

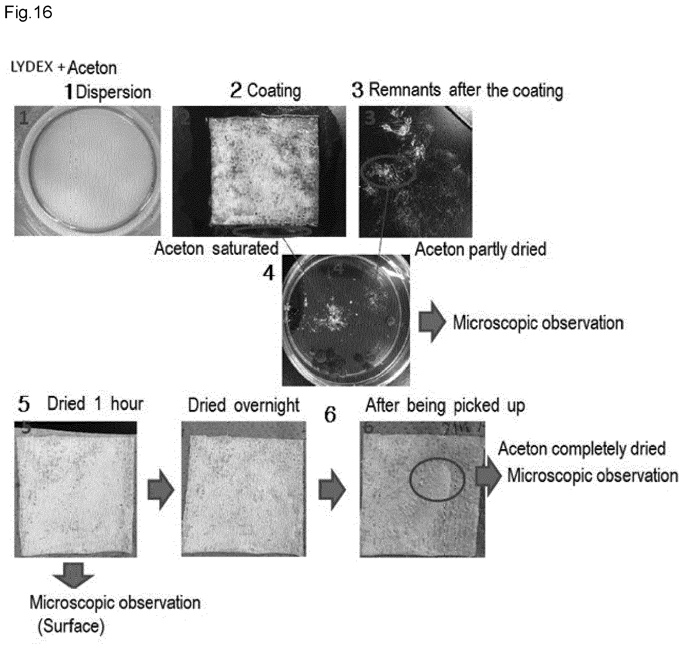

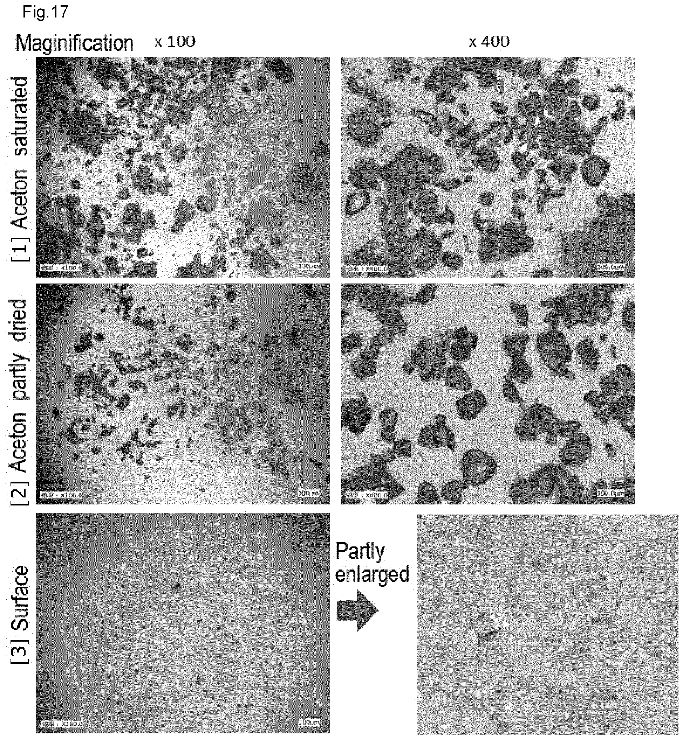

The procedures here were exactly same as in the above Reference Example 1. The situation at each stage is shown in a set of photographs of Fig.16 similar to the set of photographs of Fig.13 for Reference Example 1. Further, Fig.17 shows a series of micrographs obtained in exactly the same manner as in Fig.14 for the Reference Example 1. However, since the adhesive resin layer was not formed, the cut surface was not observed. Then, in a pair of photographs on left and right sides, of Fig.18, the dry powder (left photo) obtained after a series of procedures and the mixed powder of the original two-reactant adhesive (LYDEX) (right photo; "LYDEX 2.5 / 20") are shown in a state placed on a brown paper wiper ("Kimtowel 4 ply" of Nippon Paper Crecia Co., Ltd.), as observed with a stereomicroscope in the same manner for those in the Fig.17.

As is clear from Fig.17, although the mixed powder of the two-reactant adhesive (LYDEX) was able to be dispersed in acetone, but it was never observed in acetone the partial fusing, which was the case in the absolute ethanol.

And, the mixed powder remained as flowable even after partially dried. Thus, the formation of the resin layer by the bonding between the powders did not occur. Further, as is clear from Fig. 18, no clear change was observed in the shape of the mixed powder of the two-reactant adhesive (LYDEX) even after being dispersed in acetone and then dried.

<Reference example 3> 2% hydrous acetone

By adding distilled water to the above acetone (Wako 1st grade), hydrous acetone having a water content of 2% was prepared. Then, using this 2% hydrous acetone as a dispersion medium, the adhesive powder is dispersed in the dispersion medium and applied or "fixed" onto the base sheet, and then dried, in the same manner as in Example 1 and Reference Examples 1 and 2 except for the following --- the size of the paper wiper sheet was halved to 2.5 × 5 cm, and the amount of the two-reactant adhesive and the amount of the dispersion medium were also halved. Thus, 0.25 g of the mixed powder ("LYDEX2.5 / 20") of the two-reactant adhesive (LYDEX) used in Example 1 and so on, was dispersed in 3 mL (2.4g) of 2% hydrous acetone in exactly the same procedures as in Example 1. Then, the adhesive sheet obtained after application and drying was picked up with tweezers.

Appearances of the above stages are shown in a series of photographs in FIG.19. Further, Fig.20 shows, in a pair of left and right photographs, the surface of the adhesive sheet after drying, similarly to the bottom parts of Figs. 14 and 17. The photo on the right side is a partial enlargement of the photo on the left side. As shown in a series of photographs of Fig.19, especially in the photograph on the right end, it was able to fix the adhesive resin onto the base sheet when 2% hydrous acetone is used, unlike the case where non-hydrous acetone is used (Reference Example 2). In other word, one continuous adhesive resin layer was able be formed on the base sheet. Further, as is clear when the photographs of the surface of Fig.20 are compared with the bottom part of Fig.17, the particles (grains) are in close contact with each other.

<Reference Example 4> Non-reactive similar mixed powder

Here, the aldehyded dextran (first reactant; AD) was replaced by the powder of the above-mentioned dextran ("Dextran 70 (Dex-70)" of Meito Sangyo Co., Ltd.), which was used as raw material for the aldehyded dextran. The powder of this dextran and the powder of the succinic anhydride-treated polylysine (second reactant; SAPL) same as used in Example 1 were mixed at a weight ratio of 4/1 in the same manner as above. Using the non-reactive similar mixed powder ("Dex-70 + SAPL") thus obtained, an adhesive sheet was prepared by the same procedures as in the above Reference Examples 1 and 2.

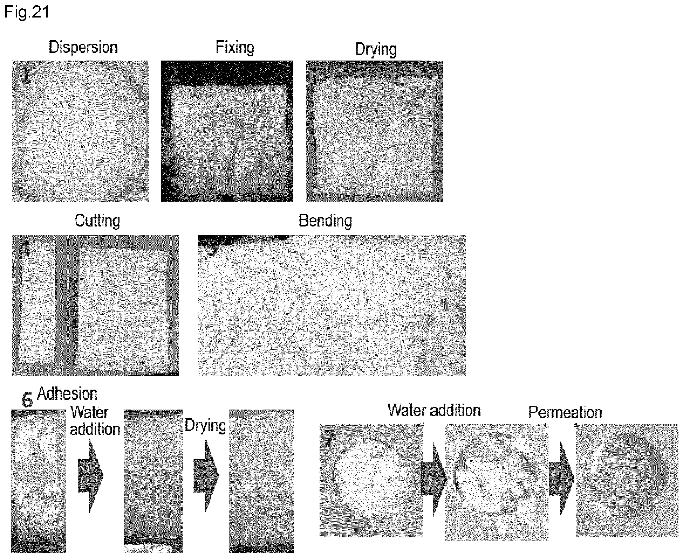

In detail, first, in the same procedure as in Example 1, 0.5 g of the non-reactive similar mixed powder ("Dex-70 + SAPL") was added to 6 mL (4.9 g) of "anhydrous" ethanol (99.5 vol% or more, Wako first grade) and uniformly dispersed by ultrasonic stirring. Then, this dispersion was applied to a sheet of paper wiper (5 × 5 cm of "Kimwipe S-200") in exactly the same manner as above and dried. As shown in the top right photograph of Fig. 21, the resin layer was able to be formed and fixed on one side (front or back) of the sheet of the paper wiper.

When the dried resin layer coated sheet was cut into three equal-size parts and then wrapped around the arm to be bent, cracks occurred in the resin layer as shown in a photograph of Fig.21, on its right-side in middle rank. However, no shedding or peeling off was observed in the resin layer or for the adhesive powder (grains). In this state, the entire resin layer coated sheet was wetted by spraying the physiological saline solution. Then, as shown in Fig. 21, on its bottom in left-side half, the sheet adhered to the skin surface of the arm. However, of course, gelation did not occur.

On the other hand, as shown in Fig.21, on its bottom in right-side half, when the above-mentioned non-reactive similar mixed powder ("Dex-70 + SAPL") was placed in a petri dish and added with water, then, as a whole, became sticky, uniform and transparent liquid.

<Reference Example 5> Modified polylysine (second reactant; SAPL) only

A resin-coated sheet was prepared using only the powder of modified polylysine (succinic anhydride-treated polylysine) by procedures same as in Reference Examples 1 and 2. That is, a resin-coated sheet was prepared using the modified polylysine (residual amino group ratio 84.7%) powder same as used in each of the above Examples and Reference Examples. Specifically, in the procedures same as in Example 1, 0.5 g of modified polylysine (SAPL) powder was added to 6 mL (4.9 g) of "anhydrous" ethanol (99.5 vol% or more, Wako 1st grade); and then was uniformly dispersed by ultrasonic stirring. Subsequently, such dispersion was applied to a sheet of paper wiper (5 × 5 cm of "Kimwipe S-200") in exactly the same manner and dried.

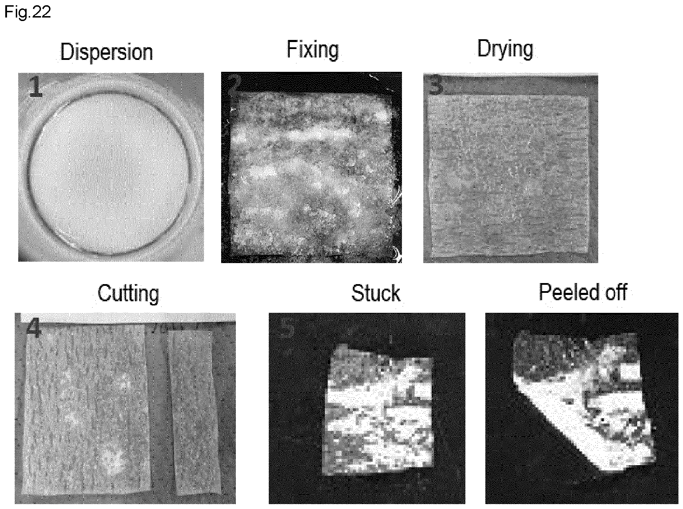

As shown in a series of photographs in top part of Fig. 22, a colorless and transparent resin layer was formed as the ethanol as a dispersion medium evaporated from the dispersion liquid, which had been applied to the sheet of the paper wiper. After being completely dried, the formed resin layer-coated sheet had the appearance of two-layer sheet formed by laminating a transparent resin film on the sheet of the paper wiper. The resin layer-coated sheet thus obtained was cut out with scissors to a size of less than one-third of original size and placed on a top plate of a laboratory table made of a melamine decorative plate. Then, the entire resin layer forming sheet was wetted by spraying the physiological saline solution. Then, the resin layer-coated sheet was got stuck to the top plate of the laboratory table. However, after a few hours, as the drying progressed, the sheet spontaneously peeled off from the top plate of the laboratory table.

From the results obtained from the above reference examples (Figs. 13 to 22), it was presumed as follows.

(i) The formation of the adhesive resin layer on the porous base sheet is due to the partial fusing of the modified polylysine (SAPL) powder in absolute ethanol or in a dispersion medium equivalent to the absolute ethanol.

(ii) Therefore, the formation of the adhesive resin layer on the base sheet does not require any chemical reaction between the aldehyde group and the amine group, that is, the reaction for hydrogel formation.

(iii) The aldehyded dextran (AD) powder itself hardly changes in shape in absolute ethanol or an equivalent dispersion medium.

(iv) In the adhesive resin layer on the base sheet, grains (granular portions) derived from the powder of aldehyde dextran (AD) are linked to each other, and fixed to the base sheet, presumably through a continuous layer derived from the modified polylysine (SAPL). In view of the structure of such an adhesive resin layer, even when the reaction between the aldehyde group and the amine group occurs, it would be limited to the surface of the grains (granular portions) derived from the aldehyded dextran (AD) powder.

(v) As the base sheet, a textile product sheet having a thickness of about 30 to 70 $\mu$m is sufficient. As the textile sheet, those having a porous structure equivalent to that of a paper wiper made of only wood pulp are enough. In other word, the fiber sheet product having such an extent of thickness and a porous structure is able to be used to fix the adhesive resin layer and to reinforce the tissues in a required extent.

Industrial applicability

The sheet-shaped tissue reinforcement of the present invention has a high tissue reinforcing effect and is able to be used as a hemostatic material for surgery and/or trauma and as a prevention of air leakage on the lung excision surface, and is thus able to be used, for example, in the medical product manufacturing industry.

**Claims**

1. A sheet-shaped tissue adhesive/reinforcement formed of:

    a base sheet having biodegradability and porous structure, and
    an adhesive resin layer fixed and formed on the base sheet,
    the adhesive resin layer formed of;
    first reactant formed of aldehyded glycan, and
    second reactant formed of partially carboxylated polylysine,
    the adhesive resin layer having:

        a structure of granules derived from powder of the first reactant and
        a connecting layer derived from the second reactant, which connects the granules with each other and fixes each of the granules onto the base sheet.

2. The sheet-shaped tissue adhesive/reinforcement according to claim 1, wherein the base sheet is non-woven fabric, woven fabric, knitted fabric, mesh sheet, sponge sheet, or other continuous porous sheet, and has a thickness of 15 $\mu$m to 500 $\mu$m; and
the adhesive resin layer has a thickness of 100 $\mu$m to 800 $\mu$m.

3. The sheet-shaped tissue adhesive/reinforcement according to claim 1 or 2, wherein the granules derived from the powder of the first reactant has an average particle size of 20 to 100 $\mu$m.

4. The sheet-shaped tissue adhesive/reinforcement according to any one of claims 1 to 3, wherein the aldehyded glycan comprising the first reactant, has 0.2 to 0.5 aldehyde groups per monosaccharide unit; and
the partially carboxylated polylysine comprising the second reactant has a residual amino group ratio of 70 to 93%.

5. The sheet-shaped tissue adhesive/reinforcement according to any one of claims 1 to 4, wherein the adhesive resin layer contains thrombin, hemocoagulase, or other blood coagulation promoter.

6.  The sheet-shaped tissue adhesive/reinforcement according to any one of claims 1 to 5, the adhesive resin layer containing, based on the total weight of the first reactant and the second reactant 5 to 30% by weight of: hydroxypropyl cellulose (HPC), hydroxypropyl methylcellulose (HPMC), or collagen or a derivative or other biodegradable polymer; and/or 1 to 25% by weight of glycerin or other moisturizing component.

7.  A method for producing a sheet-shaped tissue adhesive/reinforcement according to any one of claims 1-6, comprising:

    preparing a base sheet having biodegradability and porous structure,
    a powder of the first reactant comprising aldehyded glycan,
    a powder of the second reactant comprising partially carboxylated polylysine, and
    ethanol having a water content of less than 2% as dispersion medium;
    saturating at least the powder of the second reactant with the dispersion medium and causing partial fusing of the powder of the second reactant so as to form on the base sheet, a layer containing the first reactant, the second reactant and the dispersion medium; and
    removing the dispersion medium from the layer to form an adhesive resin layer containing the first reactant and the second reactant, as fixed on the base sheet.

8.  The method according to claim 7, comprising: preparing a mixed powder of the first reactant and the second reactant, and adopting any of (i) to (iv) below or any combination thereof so as to form the layer containing the mixed powder and the dispersion liquid on the base sheet and to subsequently remove the dispersion medium:

    (i) preparing a dispersion liquid, in which the mixed powder is dispersed in the dispersion medium, and discharging this dispersion to be applied onto the base sheet.
    (ii) preparing a dispersion liquid, in which the mixed powder is dispersed in the dispersion medium, then preparing a layer of the dispersion medium as spread on a surface and abutting the base sheet onto the layer so as to be coated with the dispersion;
    (iii) coating the base sheet with the mixed powder, and then with the dispersion medium or saturating the base sheet with the dispersion medium;
    (iv) saturating the base sheet with the dispersion medium, and then coating the base sheet with the mixed powder.

9.  The method according to claim 7 or 8, comprising adopting, for the coating with the mixed powder by the above (iii) or (iv), a powder coating device, which comprises:

    a powder discharging portion, in a way of dropping powder from a mesh openings or multiple small holes, or by way of a spray gun or dispenser; and
    a drive mechanism that continuously or sequentially moves the relative position of the base sheet with respect to the powder discharge portion.

10. The method according to any one of claims 7 to 9, comprising adding to the dispersion medium, to a mixed powder of the first reactant and the second reactant, or to the powder of the first reactant or the second reactant, with: hydroxypropyl cellulose (HPC), hydroxypropyl methylcellulose (HPMC), collagen or derivatives thereof, or other biodegradable polymers that is able to be dissolved or dispersed in a dispersion medium; and/or glycerin or other moisturizing ingredients.

11. The method according to any one of claims 7 to 10, comprising adding to the dispersion medium, to a mixed powder of the first reactant and the second reactant, or to the powder of the first reactant or the second reactant, with: thrombin, hemocoagulase, or other blood coagulation promoter.

12. A sheet-shaped tissue adhesive/reinforcement according to any of claims 1 to 6, wherein the adhesive resin layer forms a coating layer; and
    the coating layer is formed of the first reactant and the second reactant.

13. A method for producing the sheet-shaped tissue adhesive/reinforcement according to claim 7, comprising:
    preparing a mixed powder of the first reactant and the second reactant, the base sheet and low-water content ethanol having water content of 1.0% or less; forming the coating layer by coating the base sheet with dispersion liquid, which is obtained by dispersing the mixed powder in the low-water content ethanol and by subsequent drying.

**Patentansprüche**

1. Blattförmiges Gewebe-Klebstoff-Nerstärkungsmaterial, das gebildet wird aus:

   einem Basisblatt mit biologischer Abbaubarkeit und poröser Struktur und
   einer klebenden Harzschicht, die auf dem Basisblatt befestigt und ausgebildet ist,
   wobei die klebende Harzschicht gebildet wird aus;
   einem ersten Reaktanten, der aus aldehydiertem Glykan gebildet wird, und
   einem zweiten Reaktanten, der aus teilweise carboxyliertem Polylysin gebildet wird,
   wobei die klebende Harzschicht aufweist:

   eine Struktur von Körnchen, die aus dem Pulver des ersten Reaktanten abgleitet werden, und
   eine Verbindungsschicht, die aus dem zweiten Reaktanten abgeleitet wird die Körnchen miteinander verbindet und jedes der Körnchen auf dem Basisblatt fixiert.

2. Blattförmiges Gewebe-Klebstoff-/Verstärkungsmaterial nach Anspruch 1, wobei das Basisblatt ein Vlies, ein Gewebe, ein Gewirke, ein Maschenblatt, ein Schwammblatt oder ein anderes kontinuierliches poröses Blatt ist und eine
   Dicke von 15 $\mu$m bis 500 $\mu$m aufweist; und die klebende Harzschicht eine Dicke von 100 $\mu$m bis 800 $\mu$m aufweist.

3. Blattförmiges Gewebe-Klebstoff-/Verstärkungsmaterial nach Anspruch 1 oder 2, wobei das aus dem Pulver des
   ersten Reaktanten abgeleiteten Körnchen eine durchschnittliche Teilchengröße von 20 bis 100 $\mu$m aufweisen.

4. Blattförmiges Gewebe-Klebstoff-/Verstärkungsmaterial nach einem der Ansprüche 1 bis 3, wobei das aldehydierte
   Glykan, das den ersten Reaktanten aufweist, 0,2 bis 0,5 Aldehydgruppen pro Monosaccharideinheit hat; und das
   teilweise carboxylierte Polylysin, das den zweiten Reaktanten aufweist, einen Restaminogruppenanteil von 70 bis
   93 % hat.

5. Blattförmiges Gewebe-Klebstoff-/Verstärkungsmaterial nach einem der Ansprüche 1 bis 4, wobei die klebende
   Harzschicht Thrombin, Hämokoagulase oder einen anderen Blutgerinnungspromotor enthält.

6. Blattförmiges Gewebe-Klebstoff-/Verstärkungsmaterial nach einem der Ansprüche 1 bis 5, wobei die klebende
   Harzschicht, bezogen auf das Gesamtgewicht des ersten Reaktanten und des zweiten Reaktanten, 5 bis 30 Gew.-
   % Hydroxypropylcellulose (HPC), Hydroxypropylmethylcellulose (HPMC) oder Kollagen oder ein Derivat oder ein
   anderes biologisch abbaubares Polymer und/oder 1 bis 25 Gew.-% Glycerin oder eine andere feuchtigkeitsspendende Komponente enthält.

7. Verfahren zum Herstellen eines blattförmigen Gewebe-Klebstoff-/Verstärkungsmaterials nach einem der Ansprüche
   1 bis 6, das aufweist:

   Vorbereiten eines Basisblatts mit biologischer Abbaubarkeit und poröser Struktur,
   eines Pulvers des ersten Reaktanten, der ein aldehydiertes Glykan aufweist,
   eines Pulvers des zweiten Reaktanten, der teilweise carboxyliertes Polylysin aufweist, und
   Ethanol mit einem Wassergehalt von weniger als 2 % als Dispersionsmedium;
   Sättigen mindestens des Pulvers des zweiten Reaktanten mit dem Dispersionsmedium und Bewirken eines
   teilweisen Schmelzens des Pulvers des zweiten Reaktanten, um auf der Basisblatt eine Schicht zu bilden, die
   den ersten Reaktanten, den zweiten Reaktanten und das Dispersionsmedium enthält; und
   Entfernen des Dispersionsmediums von der Schicht, um eine klebende Harzschicht zu bilden, die den ersten
   Reaktanten und den zweiten Reaktanten enthält, wie sie auf dem Basisblatt fixiert ist.

8. Verfahren nach Anspruch 7, das aufweist: Vorbereiten eines gemischten Pulvers aus dem ersten Reaktanten und
   dem zweiten Reaktanten, und Anwenden eines von (i) bis (iv) oder einer Kombination davon, um die Schicht, die
   das gemischte Pulver und die Dispersionsflüssigkeit enthält, auf dem Basisblatt zu bilden und anschließend das
   Dispersionsmedium zu entfernen:

   (i) Vorbereiten einer Dispersionsflüssigkeit, in der das gemischte Pulver in dem Dispersionsmedium dispergiert ist, und Abgeben dieser Dispersion, um sie auf das Basisblatt aufzutragen.

(ii) Vorbereiten einer Dispersionsflüssigkeit, in der das gemischte Pulver im Dispersionsmedium dispergiert ist, dann Vorbereiten einer Schicht des Dispersionsmediums, die auf eine Oberfläche aufgetragen wird, und Auflegen des Basisblatts auf die Schicht, so dass sie mit der Dispersion beschichtet wird;

(iii) Beschichten des Basisblatts mit dem gemischten Pulver und dann mit dem Dispersionsmedium oder Sättigen des Basisblatts mit dem Dispersionsmedium;

(iv) Sättigen des Basisblatts mit dem Dispersionsmedium und anschließend Beschichten des Basisblatts mit dem gemischten Pulver.

9. Verfahren nach Anspruch 7 oder 8, das das Anwenden zum Beschichten mit dem gemischten Pulver nach (iii) oder (iv) einer Pulverbeschichtungsvorrichtung aufweist, die aufweist:

einen Pulverabgabeabschnitt, durch Fallenlassen von Pulver aus einer Maschenöffnung oder mehrere kleine Löcher, oder durch eine Sprühpistole oder einen Spender; und

einen Antriebsmechanismus, der die relative Position des Basisblatts in Bezug auf den Pulverabgabeabschnitt kontinuierlich oder sequentiell bewegt.

10. Verfahren nach einem der Ansprüche 7 bis 9, das das Zugeben zum Dispersionsmedium, zu einem gemischten Pulver des ersten Reaktanten und des zweiten Reaktanten oder zum Pulver des ersten Reaktanten oder des zweiten Reaktanten von Hydroxypropylcellulose (HPC), Hydroxypropylmethylcellulose (HPMC), Kollagen oder Derivaten davon oder anderen biologisch abbaubaren Polymeren, die in einem Dispersionsmedium gelöst oder dispergiert werden können; und/oder Glycerin oder anderen feuchtigkeitsspendenden Zutaten aufweist.

11. Verfahren nach einem der Ansprüche 7 bis 10, das das Zugeben zum Dispersionsmedium, zu einem gemischten Pulver des ersten Reaktanten und des zweiten Reaktanten oder zum Pulver des ersten Reaktanten oder des zweiten Reaktanten aufweist von: Thrombin, Hämokoagulase oder ein anderer Blutgerinnungsförderer.

12. Blattförmiges Gewebe-Klebstoff-/Verstärkungsmaterial nach einem der Ansprüche 1 bis 6, wobei die klebende Harzschicht eine Beschichtungsschicht bildet; und die Beschichtungsschicht aus dem ersten Reaktanten und dem zweiten Reaktanten gebildet wird.

13. Verfahren zum Herstellen des blattförmigen Gewebe-Klebstoff-/Verstärkungsmaterials nach Anspruch 7, das aufweist:

Vorbereiten eines gemischten Pulvers aus dem ersten Reaktanten und dem zweiten Reaktanten, des Basisblatts und Ethanols mit niedrigem Wassergehalt, das einen Wassergehalt von 1,0 % oder weniger aufweist;

Bilden der Beschichtungsschicht durch Beschichten des Basisblatts mit einer Dispersionsflüssigkeit, die durch Dispergieren des gemischten Pulvers in dem Ethanol mit niedrigem Wassergehalt und durch anschließendes Trocknen erhalten wird.

## Revendications

1. Matière de renforcement/adhésif tissulaire en forme de feuille constituée :

d'une feuille de base dotée de biodégradabilité et d'une structure poreuse, et
d'une couche de résine adhésive fixée et formée sur la feuille de base, la couche de résine adhésive étant constituée :

d'un premier réactif formé d'un glycane aldéhydique, et
d'un deuxième réactif formé de polylysine partiellement carboxylée,
la couche de résine adhésive présentant :

une structure de granules dérivée de la poudre du premier réactif et
une couche de liaison dérivée du deuxième réactif, reliant les granules entre eux et fixant chacun des granules sur la feuille de base.

2. Matière de renforcement/adhésif tissulaire en forme de feuille selon la revendication 1, où la feuille de base est un textile non tissé, un textile tissé, un textile tricoté, une feuille à mailles, une feuille d'éponge ou une autre feuille poreuse continue, et a une épaisseur comprise entre 15 $\mu$m et 500 $\mu$m ; et où la couche de résine adhésive a une épaisseur comprise entre 100 $\mu$m et 800 $\mu$m.

3. Matière de renforcement/adhésif tissulaire en forme de feuille selon la revendication 1 ou la revendication 2, où les granules issus de la poudre du premier réactif ont une granulométrie moyenne de 20 à 100 $\mu$m.

4. Matière de renforcement/adhésif tissulaire en forme de feuille selon l'une des revendications 1 à 3, où le glycane aldéhydique comprenant le premier réactif, comporte 0,2 à 0,5 groupes aldéhydiques par unité monosaccharidique ; et où la polylysine partiellement carboxylée comprenant le deuxième réactif a une teneur de groupe aminé résiduel comprise entre 70 et 93 %.

5. Matière de renforcement/adhésif tissulaire en forme de feuille selon l'une des revendications 1 à 4, où la couche de résine adhésive contient de la thrombine, de l'hémocoagulase ou un autre promoteur de coagulation sanguine.

6. Matière de renforcement/adhésif tissulaire en forme de feuille selon l'une des revendications 1 à 5, où la couche de résine adhésive contient, par rapport au poids total du premier réactif et du deuxième réactif, 5 à 30 % en poids : d'hydroxypropylcellulose (HPC), d'hydroxypropylméthylcellulose (HPMC), ou de collagène, ou d'un dérivé, ou d'un autre polymère biodégradable ; et/ou 1 à 25 % en poids de glycérine ou d'un autre composant hydratant.

7. Procédé de fabrication d'une matière de renforcement/adhésif tissulaire en forme de feuille selon l'une des revendications 1 à 6, comprenant :

la préparation d'une feuille de base dotée de biodégradabilité et d'une structure poreuse,
une poudre du premier réactif comprenant un glycane aldéhydique,
une poudre du deuxième réactif comprenant de la polylysine partiellement carboxylée, et
de l'éthanol ayant une teneur en eau inférieure à 2 % comme milieu de dispersion ;
la saturation au moins de la poudre du deuxième réactif avec le milieu de dispersion et la génération d'une fusion partielle de la poudre du deuxième réactif de manière à former sur la feuille de base une couche contenant le premier réactif, le deuxième réactif et le milieu de dispersion ; et
l'élimination du milieu de dispersion de la couche de manière à former une couche de résine adhésive contenant le premier réactif et le deuxième réactif, telle que fixée sur la feuille de base.

8. Procédé selon la revendication 7, comprenant : la préparation d'une poudre mélangée du premier réactif et du deuxième réactif, et l'application d'une des étapes (i) à (iv) ci-dessous ou d'une combinaison quelconque de celles-ci de manière à former la couche contenant la poudre mélangée et le liquide de dispersion sur la feuille de base et à éliminer ensuite le milieu de dispersion :

(i) préparation d'un liquide de dispersion où la poudre mélangée est dispersée dans le liquide de dispersion, et déversement de cette dispersion pour application sur la feuille de base.
(ii) préparation d'un liquide de dispersion où la poudre mélangée est dispersée dans le milieu de dispersion, puis préparation d'une couche du milieu de dispersion à étaler sur une surface et mise en place de la feuille de base sur la couche de manière à la recouvrir de la dispersion ;
(iii) revêtement de la feuille de base avec la poudre mélangée, puis avec le milieu de dispersion ou saturation de la feuille de base avec le milieu de dispersion ;
(iv) saturation de la feuille de base avec le milieu de dispersion, puis revêtement de la feuille de base avec la poudre mélangée.

9. Procédé selon la revendication 7 ou la revendication 8, comprenant la sélection, pour le revêtement avec la poudre mélangée lors des étapes (iii) ou (iv) ci-dessus, d'un dispositif de revêtement de poudre, comprenant :
une partie de déversement de poudre, de manière à faire tomber la poudre d'ouvertures de maille ou d'une pluralité de petits trous, ou au moyen d'un pistolet de pulvérisation ou d'un diffuseur ; et un mécanisme d'entraînement déplaçant de manière continue ou séquentielle la position relative de la feuille de base par rapport à la partie de déversement de poudre.

10. Procédé selon l'une des revendications 7 à 9, comprenant l'ajout au milieu de dispersion, à une poudre mélangée du premier réactif et du deuxième réactif, ou à la poudre du premier réactif ou du deuxième réactif : d'hydroxypropylcellulose (HPC), d'hydroxypropylméthylcellulose (HPMC), de collagène ou de ses dérivés, ou d'autres polymères biodégradables pouvant être dissous ou dispersés dans un milieu de dispersion ; et/ou de glycérine ou d'autres ingrédients hydratants.

11. Procédé selon l'une des revendications 7 à 10, comprenant l'ajout au milieu de dispersion, à une poudre mélangée du premier réactif et du deuxième réactif, ou à la poudre du premier réactif ou du deuxième réactif : de thrombine, d'hémocoagulase, ou d'autres promoteurs de coagulation sanguine.

12. Matière de renforcement/adhésif tissulaire en forme de feuille selon l'une des revendications 1 à 6, où la couche de résine adhésive forme une couche de revêtement ; et où la couche de revêtement est constituée du premier réactif et du deuxième réactif.

13. Procédé de fabrication de la matière de renforcement/adhésif tissulaire en forme de feuille selon la revendication 7, comprenant :
la préparation d'une poudre mélangée du premier réactif et du deuxième réactif, de la feuille de base et de l'éthanol à faible teneur en eau ayant une teneur en eau de 1,0 % ou moins ; la formation de la couche de revêtement par recouvrement de la feuille de base avec le liquide de dispersion, obtenu en dispersant la poudre mélangée dans l'éthanol à faible teneur en eau, et par séchage consécutif.

Fig.01

| 10% Water content Ethanol | 5% Water content Ethanol | 2% Water content Ethanol | 1% Water content Ethanol | Water content$\leqq$0.5 Ethanol |

| Not-dispersed | Not-dispersed | Partly dispersed | Partly dispersed | Dispersed |

Fig.02

Air-leak sites
If fewer than 4, then punctured to
be formed with a syringe needle

Cut section

Lateral left lobe

Guideline for resecting

3mm X 3mm sheet-shaped Lydex was
pasted and attached to the air-leak sites

Or

Lydex powder was sprayed
on the cut surface

Fig.03

## PLLA dese mesh

## PLLA coarse mesh

Fig.04

Coating with the dispersion liquid

Side of the adhesive resin layer

Side of the base sheet

Drying

Fig.05

Coating with the dispersion liquid

Side of the adhesive resin layer

Side of the base sheet

Drying

Fig.06

5.0

1.25   3.75   cm

Marking position

Adhesion area

Sheet or collagen casing

Collagen casing

Fixing with jigs   Fixing with jigs   Pulling

Sheet-shaped Lydex
(PLLA mesh material)

Fig.07

Fig.08

( N )

p < 0.01

BOLHEAL          LYDEX sheet

Fig.09

Fig.10

Enlarged

Fig.11

# LYDEX

Rolled in long direction

Unrolled after rolled

Coming-off site as enlarged

Powder slightly came off

Fig.12

# 20%HPC-LYDEX

Rolled
in long direction

Unrolled after rolled

Powder very slightly came off

Fig.13

1 Dispersion     2 Coating     3 Remnants after the coating

EtOH saturated         EtOH partly dried

4

→ Microscopic observation

5 Dried 1 hour     6 Completely dried

Microscopic observation
(Surface)

Microscopic observation
(Cross section)

Fig.14

Magnification    x 100                                    x 400

[1] EtOH saturated

[2] EtOH partly dried

Presumably,
SAPL fused part

[3] Surface

Partly
enlarged

Fig.15

Magnification    x 100    Thickness measurement for base sheet

[4] Cross section

Base sheet thickness
(Microscopic observation)

53 μm

39 μm

About 30 to 70 μm
by micrometer
measurement

Fig.16

LYDEX + Aceton

1 Dispersion     2 Coating     3 Remnants after the coating

Aceton saturated     Aceton partly dried

4     Microscopic observation

5 Dried 1 hour     Dried overnight   6   After being picked up

Aceton completely dried
Microscopic observation

Microscopic observation
(Surface)

Fig.17

Maginification  x 100                              x 400

[1] Aceton saturated

[2] Aceton partly dried

[3] Surface

Partly
enlarged

Fig.18

[4] Aceton completely dried          Ordinary LYDEX

Fig.19

LYDEX + 98% Aceton (water content of 2%)

Dispersion      Fixing      Drying      Picked up

Fig.20

Sheet surface x100

Partly enlarged

Magnification

Fig.21

Dispersion | Fixing | Drying

Cutting | Bending

6 Adhesion — Water addition — Drying

Water addition — Permeation

Fig.22

Dispersion | Fixing | Drying

Cutting | Stuck | Peeled off

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008066182 A
- JP 4571693 B
- JP 1986S61034830 B
- WO 2012029971 A
- JP 2017192560 A
- JP 2017222915 A
- JP 4681214 B
- JP 2016063919 A

**Non-patent literature cited in the description**

- Development of new biodegradable hydrogel glue for preventing alveolar air leakage. *J. Thoracic and Cardiovascular Surgery,* November 2007, vol. 134 (5), 1241-8